(19) Europäisches Patentamt
European Patent Office
Office européen des brevets

(11) EP 1 981 876 B1

(12) **EUROPEAN PATENT SPECIFICATION**

(45) Date of publication and mention of the grant of the patent:
**04.05.2011 Bulletin 2011/18**

(21) Application number: **07704895.7**

(22) Date of filing: **11.01.2007**

(51) Int Cl.:
***C07D 401/14*** (2006.01) ***C07D 403/12*** (2006.01)
***C07D 403/14*** (2006.01) ***C07D 405/14*** (2006.01)
***C07D 409/14*** (2006.01) ***A61K 31/505*** (2006.01)
***A61P 31/14*** (2006.01)

(86) International application number:
**PCT/GB2007/000065**

(87) International publication number:
**WO 2007/080401 (19.07.2007 Gazette 2007/29)**

(54) **TRIAZOLOANILINOPYRIMIDINE DERIVATIVES FOR USE AS ANTIVIRAL AGENTS**

TRIAZOLOANILINOPYRIMIDINDERIVATE ZUR VERWENDUNG ALS ANTIVIRALE MITTEL

DÉRIVÉS DE TRIAZOLOANILINOPYRIMIDINE POUVANT ÊTRE EMPLOYÉS EN TANT QU'AGENTS ANTIVIRAUX

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **11.01.2006 GB 0600510**
**19.06.2006 GB 0612116**

(43) Date of publication of application:
**22.10.2008 Bulletin 2008/43**

(73) Proprietor: **Arrow Therapeutics Limited**
**London SE1 1DA (GB)**

(72) Inventors:
- **MATHEWS, Neil**
**SE1 1DA (GB)**
- **THOMAS, Alexander, James, Floyd**
**SE1 1DA (GB)**
- **SPENCER, Keith, Charles**
**London SE1 1DA (GB)**
- **TIBERGHIEN, Nathalie**
**London SE1 1DA (GB)**
- **PILKINGTON, Christopher, John**
**SE1 1DA (GB)**
- **JENNENS, Lyn**
**London SE1 1DA (GB)**
- **CHANA, Surinder**
**London SE1 1DA (GB)**
- **FRASER, Ian, John**
**London SE1 1DA (GB)**

(56) References cited:
**WO-A-2005/105761** **WO-A-2006/079833**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 981 876 B1

## Description

The present invention relates to a series of quinazoline derivatives which are useful in treating or preventing a flaviviridae infection.

Viruses of the family flaviviridae are small, icosahedral, enveloped viruses that contain a positive-sense RNA genome. The family consists of three genera, flavivirus, pestivirus and hepacivirus.

Many of the flaviviridae viruses are important human pathogens. Indeed, the hepacivirus genus includes the hepatitis C virus. However, there exists, as yet, no effective and safe treatment for flaviviridae infections.

WO 98/02434 discloses quinazolines as protein tyrosine kinase inhibitors. None of the compounds specifically disclosed in that document carry a triazolylaniline group at the 6- position.

WO 05/105761 discloses morpholinylanilinoquinazoline derivatives which were found to be active in inhibiting the replication of flaviviridae viruses.

It has now surprisingly been found that the quinazoline derivatives of the formula (Ib) and (Ic) are active in inhibiting replication of flaviviridae viruses and are therefore effective in treating or preventing a flaviviridae infection. The present invention therefore provides a quinazoline derivative of formula (Ib), or a pharmaceutically acceptable salt thereof:

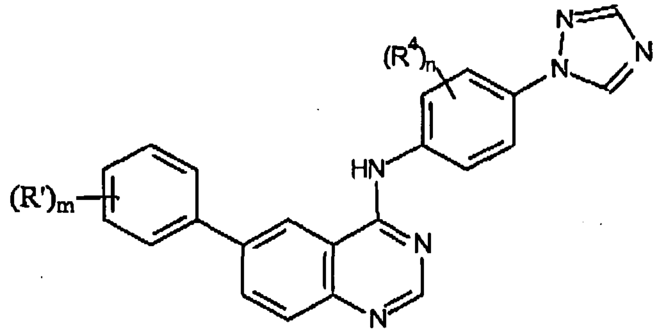

(Ib)

wherein:

- each $R^4$ is the same or different and represents halogen, $C_{1-4}$ alkyl or $C_{1-4}$ alkoxy;
- n is 0, 1 or 2;
- each R' is the same or different and represents halogen, $C_{1-4}$ alkyl or $C_{1-4}$ alkoxy; and
- m is 0, 1 or 2.

The present invention also provides a quinazoline derivative of the formula (Ic), or a pharmaceutically acceptable salt thereof:

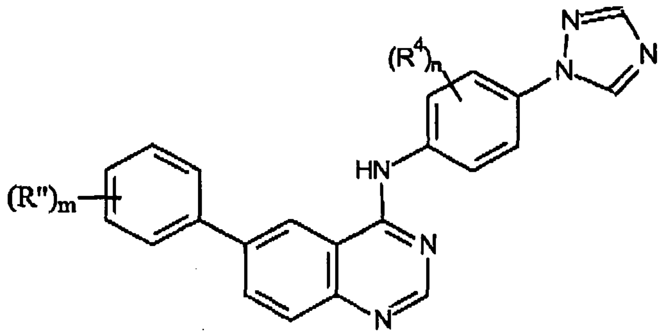

(Ic)

wherein:

- each $R^4$ is the same or different and represents halogen, $C_{1-4}$ alkyl or $C_{1-4}$ alkoxy;
- m is 1;
- n is 0, 1 or 2; and
- R" is -A', -Het-A', -L-A', -Het-L-A', -L-Het-A', -Het-L-Het'-A' or -Het-L-Het'-L' wherein:

- A' represents a phenyl, 5- to 10-membered heteroaryl, 5- to 10-membered heterocyclyl or $C_{3-6}$ carbocyclyl group which is optionally fused to a further phenyl, 5- to 10-membered heteroaryl, 5- to 10-membered heterocyclyl or $C_{3-6}$ carbocyclyl group;
- each Het and Het' is the same or different and represents -O-, -S- or -NR'-where R' is hydrogen or $C_{1-4}$ alkyl;
- each L is the same or different and represents $C_{1-4}$ alkylene; and
- each L' is the same or different and represents hydrogen or a $C_{1-4}$ alkyl, $C_{1-4}$ haloalkyl, $C_{1-4}$ hydroxyalkyl or $C_{1-4}$ aminoalkyl group,

  the phenyl, heteroaryl, heterocyclyl and carbocyclyl moieties in A' being unsubstituted or substituted by 1, 2 or 3 unsubstituted substituents which are the same or different and are selected from halogen atoms and $C_{1-4}$ alkyl, $C_{1-4}$ alkoxy, $C_{1-4}$ haloalkyl, $C_{1-4}$ haloalkoxy, hydroxy, cyano, nitro and -NR'R", wherein each R' and R" is the same or different and represents hydrogen or $C_{1-4}$ alkyl.

As used herein, a $C_{1-6}$ alkyl group or moiety is a linear or branched alkyl group or moiety containing from 1 to 6 carbon atoms, for example 1 to 4 carbon atoms. Examples of $C_{1-6}$ alkyl groups and moieties include $C_{1-4}$ alkyl groups and moieties such as include methyl, ethyl, n-propyl, i-propyl, n-butyl, i-butyl and t-butyl. For the avoidance of doubt, where two alkyl moieties are present in a group, the alkyl moieties may be the same or different.

As used herein, a $C_{1-4}$ alkylene group or moiety is a linear or branched alkylene group or moiety. Examples include methylene, n-ethylene and n-propylene groups and moieties.

As used herein, a halogen is typically chlorine, fluorine, bromine or iodine and is preferably chlorine, bromine or fluorine, more preferably chlorine, fluorine or iodine.

As used herein, a $C_{1-6}$ alkoxy group is typically a said $C_{1-6}$ alkyl group attached to an oxygen atom. A haloalkyl or haloalkoxy group is typically a said alkyl or alkoxy group substituted by one or more said halogen atoms. Typically, it is substituted by 1, 2 or 3 said halogen atoms. Preferred haloalkoxy groups include alkoxy groups substituted by one or two chlorine atoms, more preferably by one chlorine atom. Particularly preferred haloalkyl groups include $-O-(CH_2)_3-Cl$. Other preferred haloalkyl and haloalkoxy groups include perhaloalkyl and perhaloalkoxy groups such as $-CX_3$ and $-OCX_3$ wherein X is a said halogen atom, for example chlorine and fluorine. Particularly preferred haloalkyl groups are $-CF_3$ and $-CCl_3$.

As used herein, a $C_{1-4}$ hydroxyalkyl group is a $C_{1-4}$ alkyl group substituted by one or more hydroxy groups. Typically, it is substituted by one, two or three hydroxy groups. Preferably, it is substituted by a single hydroxy group. A preferred hydroxyalkyl group is $-(CH_2)_2-OH$.

As used herein, a $C_{1-4}$ aminoalkyl group is a $C_{1-4}$ alkyl group substituted by one or more -NR'R" groups wherein each R' and R" is the same or different and represents hydrogen or $C_{1-4}$ alkyl. Typically it is substituted by one, two or three -NR'R" groups wherein each R' and R" is the same or different and represents hydrogen or $C_{1-4}$ alkyl. Preferably each R' and R" is the same or different and represents hydrogen or $C_{1-2}$ alkyl, more preferably hydrogen or methyl. Preferably the $C_{1-4}$ alkyl group is substituted by a single -NR'R" group as defined above. More preferably, the $C_{1-4}$ alkyl group is substituted by a single group $-N(CH_3)_2$.

As used herein, a 5- to 10- membered heteroaryl group or moiety is a monocyclic 5- to 10- membered aromatic ring, such as a 5- or 6- membered ring, containing at least one heteroatom, for example 1, 2 or 3 heteroatoms, selected from O, S and N. Examples include pyridyl, pyrazinyl, pyrimidinyl, pyridazinyl, furanyl, thienyl, pyrazolidinyl, pyrrolyl, oxadiazolyl, oxazolyl, isoxazolyl, thiazolyl, thiadiazolyl, imidazolyl and pyrazolyl groups. Furanyl, thienyl, pyridyl and pyrimidyl groups are preferred.

As used herein, a 5- to 10- membered heterocyclyl group or moiety is a monocyclic non-aromatic, saturated or unsaturated $C_5$-$C_{10}$ carbocyclic ring in which one or more, for example 1, 2 or 3, of the carbon atoms are replaced with a moiety selected from N, O, S, S(O) and $S(O)_2$. Typically, it is a 5- to 6- membered ring.

Suitable heterocyclyl groups and moieties include pyrazolidinyl, piperidyl, piperazinyl, thiomorpholinyl, S-oxo-thiomorpholinyl, S,S-dioxo-thiomorpholinyl, morpholinyl, pyrrolidinyl, pyrrolinyl, imidazolidinyl, imidazolinyl, 1,3-dioxolanyl, 1,4-dioxolanyl, 1,2-dioxacylohexyl, 1,3-dioxacyclohexyl, 1,4-dioxacyclohexyl and pyrazolinyl groups and moieties. Preferred heterocyclyl groups are piperazinyl, pyrrolidinyl, morpholinyl and 1,4-dioxacyclohexane-groups, in particular morpholinyl and 1,4-dioxacyclohexane groups.

For the avoidance of doubt, although the above definitions of heteroaryl and heterocyclyl groups refer to an "N" moiety which can be present in the ring, as will be evident to a skilled chemist the N atom will be protonated (or will carry a substituent as defined above) if it is attached to each of the adjacent ring atoms via a single bond.

As used herein, a $C_{3-6}$ carbocyclic moiety is a monocyclic non-aromatic saturated or unsaturated hydrocarbon ring having from 3 to 6 carbon atoms. Preferably it is a saturated hydrocarbon ring (i.e. a cycloalkyl moiety) having from 3 to 6 carbon atoms. Examples include cyclopropyl, cyclobutyl, cyclopentyl and cyclohexyl.

Particularly preferred compounds of include:

1. (6-Iodo-quinazolin-4-yl)-(4-[1,2,4]triazol-1-yl-phenyl)-amine.

2. (6-tert-Butyl-quinazolin-4-yl)-(4-[1,2,4]triazol-1-yl-phenyl)-amine.
3. [7-(3-Chloro-propoxy)-6-methoxy-quinazolin-4-yl]-(4-[1,2,4]triazol-1-yl-phenyl)-amine.
4. [6-Methoxy-7-(3-morpholin-4-yl-propoxy)-quinazolin-4-yl]-(4-[1,2,4]triazol-1-yl-phenyl)-amine.
5. [6-(3,4-Difluoro-phenyl)-quinazolin-4-yl]-(4-[1,2,4]triazol-1-yl-phenyl)-amine,
6. [6-(4-Chloro-3-fluoro-phenyl)-quinazolin-4-yl]-(4-[1,2,4]triazol-1-yl-phenyl)-amine.
7. [6-(3-Fluoro-4-methoxy-phenyl)-quinazolin-4-yl]-(4-[1,2,4]triazol-1-yl-phenyl)-amine.
8. [6-(4-Ethoxy-3-fluoro-phenyl)-quinazolin-4-yl]-(4-[1,2,4]triazol-1-yl-phenyl)-amine.
9. {6-[3-Fluoro-4-(2-methoxy-ethoxy)-phenyl]-quinazolin-4-yl}-(4-[1,2,4]triazol-1-yl-phenyl)-amine.
10. [6-(3,4-Dimethoxy-phenyl)-quinazolin-4-yl]-(4-[1,2,4]triazol-1-yl-phenyl)-amine.
11. [6-(3,4-Diethoxy-phenyl)-quinazolin-4-yl]-(4-[1,2,4]triazol-1-yl-phenyl)-amine.
12. {6-[4-(2-Dimethylamino-ethoxy)-phenyl]-quinazolin-4-yl}-(4-[1,2,4]triaaol-1-yl-phenyl)-amine.
13. {6-[4-Pyridin-4-ylmethoxy)-phenyl]-quinazolin-4-yl}-(4-[1,2,4]triazol-1-yl-phenyl)-amine.
14. [6-(4-Morpholin-4-yl-phenyl)-quinazolin-4-yl]-(4-[1,2,4]triazol-1-yl-phenyl)-amine.
15. [6-(2,3-Dihydro-benzo[1,4]dioxin-6-yl)-quinazolin-4-yl]-(4-[1,2,4]triazol-1-yl-phenyl)-amine.
16. [6-(2-Methoxy-pyrimidin-5-yl)-quinazolin-4-yl]-(4-[1,2,4]triazol-1-yl-phenyl)-amine.
17. (6-Thiophen-2-yl-quinazolin-4-yl)-(4-[1,2,4]triazol-1-yl-phenyl)-amine.
18. [6-(4-Methyl-thiophen-2-yl)-quinazolin-4-yl]-(4-[1,2,4]triazol-1-yl-phenyl)-amine.
19. (6-Furan-2-yl-quinazolin-4-yl)-(4-[1,2,4-triazol-1-yl-phenyl)-amine.
20. {6-[4-(2-Dimethylamino-ethoxy)-3-fluoro-phenyl]-quinazolin-4-yl}-(4-[1,2,4]triazol-1-yl-phenyl)-amine.
21. (6-Bromo-quinazolin-4-yl)-(4-[1,2,4]triazol-1-yl-phenyl)-amine.
22. N-(2-{2-Fluoro-4-[4-(4-[1,2,4]triazol-1-yl-phenylamino)-quinazolin-6-yl]-phenoxy}-ethyl)-N,N',N'-trimethyl-ethane-1,2-diamine.
23. [6-(3-Isopropoxy-4-methoxy-phenyl)-quinazolin-4-yl]-(4-[1,2,4]triazol-1-yl-phenyl)-amine.
24. [6-(4-Fluoro-3-isopropoxy-phenyl)-quinazolin-4-yl]-(4-[1,2,4]triazol-1-yl-phenyl)-amine.
25. {6-[3-Fluoro-4-(3-pyrrolidin-1-yl-propoxy)-phenyl]-quinazolin-4-yl}-(4-[1,2,4]triazol-1-yl-phenyl)-amine.
26. {6-[3-Fluoro-4-(3-morpholin-4-yl-propoxy)-phenyl]-quinazolin-4-yl}-(4-[1,2,4]triazol-1-yl-phenyl)-amine.
27. [6-(3,4-Dimethoxy-phenyl)-quinazolin-4-yl]-(2-methyl-4-[1,2,4]triazol-1-yl-phenyl)-amine.
28. 1-(4-{6-[3-Fluoro-4-(2-methoxy-ethoxy)-phenyl]-quinazolin-4-ylamino}-phenyl)-5-methyl-1H-[1,2,4]triazole-3-carboxylic acid.
29. 1-{4-[6-(3,4-Dimethoxy-phenyl)-quinazolin-4-yl-amino]-phenyl}-5-methyl-1H-[1,2,4]triazole-3-carboxylic acid.
30. 1-{4-[6-(3,4-Dimethoxy-phenyl)-quinazolin-4-ylamino]-phenyl}-5-methyl-1H-[1,2,4]triazole-3-carboxylic acid dimethylamide.
31. 1-(4-{6-[3-Fluoro-4-(2-methoxy-ethoxy)-phenyl]-quinzoline-4-ylamino}-phenyl)-5-methyl-1H-[1,2,4]triazole-3-carboxylic acid methylamide.
32. 1-(4-{6-[3-Fluoro-4-(2-methoxy-ethoxy)-phenyl]-quinazolin-4-ylamino}-phenyl)-5-methyl-1H-[1,2,4]triazole-3-carboxylic acid dimethylamide.
33. (6-Bromo-quinazolin-4-yl)-(2-methyl-4-[1,2,4]triazol-1-yl-phenyl)-amine.
34. [1-(4-{6-[3-Fluoro-4-(2-methoxy-ethoxy)-phenyl]-quinazolin-4-ylamino}-phenyl)-5-methyl-1H-[1,2,4]triazol-3-yl]-(4-methyl-piperazin-1-yl)-methanone.
35. 2-Methoxy-4-[4-(4-[1,2,4]triazol-1-y-phenylamino)-quinazolin-6-yl-phenol.
36. 2-Methoxy-5-[4-(4-[1,2,4]tiazol-1-yl-phenylamino)-quinazolin-6-yl]phenol.
37. {6-[3-Fluoro-4-(2-methoxy-ethoxy)-phenyl]quinazolin-4-yl}-(2-methyl-4-[1,2,4]triazol-1-yl-phenyl)-amine, and pharmaceutically acceptable salts thereof.

Compounds of formula (Ib) and (Ic) containing one or more chiral centre may be used in enantiomerically or diastereoisomerically pure form, or in the form of a mixture of isomers. For the avoidance of doubt, the compounds of formula (Ib) and (Ic) can, if desired, be used in the form of solvates. Further, for the avoidance of doubt, the compounds of the invention may be used in any tautomeric form.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as citric, fumaric, maleic, malic, ascorbic, succinic, tartaric, benzoic, acetic, methanesulphonic, ethanesulphonic, benzenesulphonic or *p*-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines.

The compounds of the invention can, for example, be prepared according to the following reaction schemes. In the schemes which follow, for reasons of clarity, the $R^4$ groups have been omitted (i.e. n is zero). Analogous compounds where n is 1 or 2 can be prepared by employing a suitably functionalized 4-triazolylaniline derivative bearing the appropriate substituents in the $R^4$ position.

Scheme 1

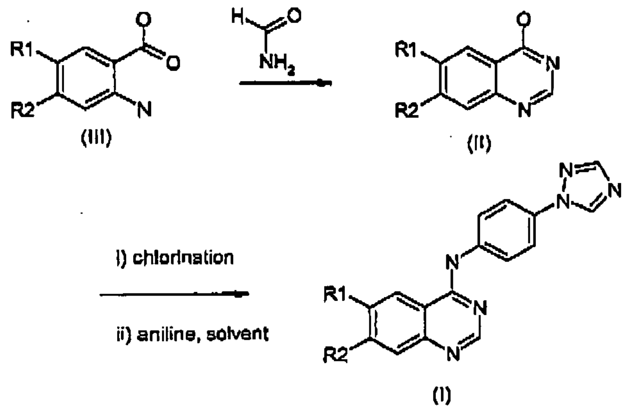


(III) (II) (I)

Referring to Scheme 1, the conversion of compounds of formula (II) to compounds of formula (I) is accomplished by converting the 4-hydroxy group of compounds of formula (II) to a suitable leaving group (e.g. chloro) using a reagent such as thionyl chloride as solvent with the addition of a catalytic activator (e.g. dimethylformamide), and subsequent reaction with 4-triazolylaniline in a suitable solvent (e.g. acetonitrile).

Referring to Scheme 1, the conversion of compounds of formula (III) to compounds of formula (II) will be well known to one skilled in the art, being conveniently performed with formamide as solvent and at elevated temperature (e.g. reflux).

Compounds of formula (I) in which $R^1$ is -A, -A-A', -A-Het-A', -A-L-A', -A-Het-L-A', -A-L-Het-A', -A-Het-L-Het'-A' or -A-Het-L-Het'-L' can alternatively be produced by the reaction shown in Scheme 2 below. The reaction is typically carried out in the presence of acetic acid at a temperature of about 120°C and for a period of about 1 hour.

Scheme 2

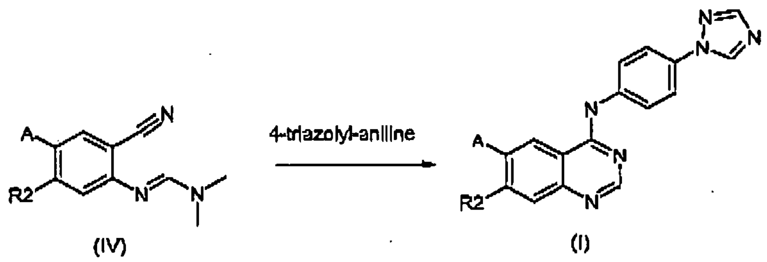


(IV) (I)

The compounds of formula (IV) used as a starting material in Scheme 2 can be prepared by one of the reactions depicted in Scheme 3 below. In Scheme 3, the groups S1 and S2 may represent protecting groups, such as benzyl, which can be replaced by the desired group by methods known in the art following reaction. Deprotection can be carried out before or after conversion of the compound of formula (IV) to the compound of formula (I).

Referring to Scheme 3, the treatment of compounds of formula (VIII) with an organometallic reagent (VII), or the treatment of compounds of formula (VI) with an organometallic reagent (V), is conveniently carried out in a suitable solvent (such as tetrahydrofuran, dimethylformamide, toluene or propan-2-ol or) and at a suitable temperature (e.g. from ambient to reflux). Conveniently, the reaction is performed under palladium catalysis (e.g. 10mol% tris (dibenzylideneacetone)dipalladium (II), 10mol% dichlorobis (triphenylphosphine)palladium (0), 1mol% bis(benzonitrile)palladium(II) chloride or 0.02 mol% tetrakis(triphenylphosphine)palladium(0)) in the presence of an organic base (e.g. triethylamine) or an inorganic base (e.g. 2N sodium carbonate or potassium phosphate). Where reagent (VII) is an organostannane (e.g. M = $SnBu_3$), one skilled in the art will recognise the reaction as an example of a Stille coupling where additional additives may be beneficial (e.g. lithium chloride), silver oxide and conveniently the reaction is performed in toluene and at reflux temperature. Where reagent (VII) is a boronic acid derivative, one skilled in the art will recognise the reaction as an example of a Suzuki-Miyaura coupling which may be conveniently performed at 60 °C in propan-2-ol.

As the skilled person in the art will appreciate, the group X in the compounds depicted in Scheme 3 is an appropriate leaving group such as I or Br.

Scheme 3

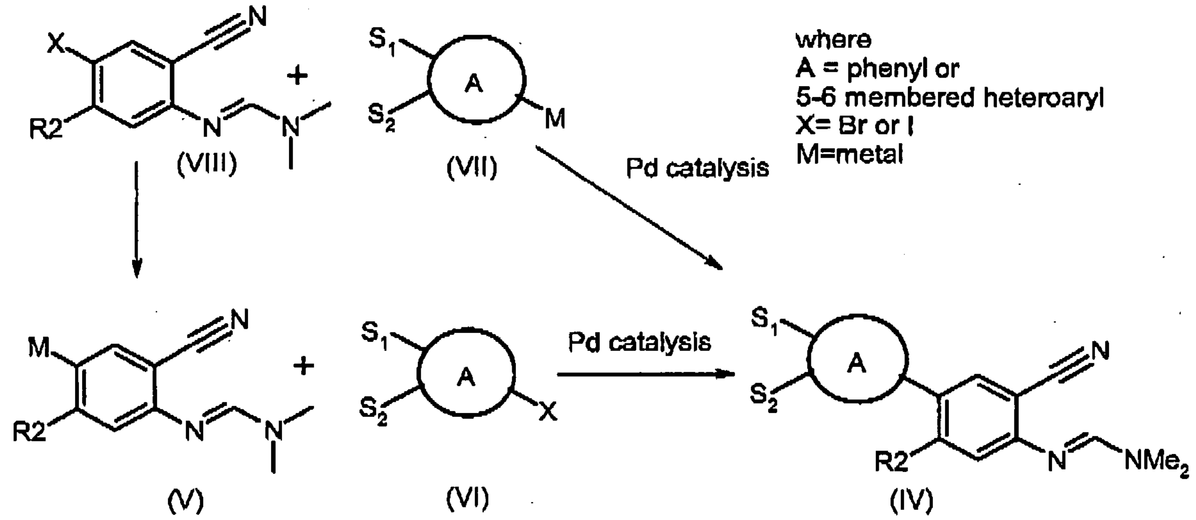


The starting materials in the above reaction scheme are known compounds, or can be prepared by analogy with known methods.

The compounds of the present invention are therapeutically useful. The present invention therefore provides a quinazoline derivative of the formula (Ia) and (Ib), as defined above, or a pharmaceutically acceptable salt thereof, for use in treating the human or animal body. Also provided is a pharmaceutical composition comprising a quinazoline derivative of the formula (Ia) and (Ib), as defined above, or a pharmaceutically acceptable salt thereof, and a pharmaceutically acceptable carrier or diluent.

Said pharmaceutical composition typically contains up to 85 wt% of a compound of the invention. More typically, it contains up to 50 wt% of a compound of the invention. Preferred pharmaceutical compositions are sterile and pyrogen free. Further, the pharmaceutical compositions provided by the invention typically contain a compound of the invention which is a substantially pure optical isomer.

As explained above, the compounds of the invention are active against a flaviviridae infection. The present invention therefore provides the use of a quinazoline derivative of the formula (Ia) and (Ib), as defined above, or a pharmaceutically acceptable salt thereof, in the manufacture of a medicament for use in treating or preventing a flaviviridae infection. Also provided is a method for treating a patient suffering from or susceptible to a flaviviridae infection, which method comprises administering to said patient an effective amount of a quinazoline derivative of formula (Ia) and (Ib) or a pharmaceutically acceptable salt thereof.

The flaviviridae family contains three genera. These are hepacivirus, flavivirus and pestivirus. The compounds of the invention are active in treating or preventing a hepacivirus infection, a flavivirus infection or a pestivirus infection.

Typical pestivirus infections which can be treated with the compounds of the invention include bovine viral diarrhea virus, classical swine fever virus and border disease virus.

Typical flavivirus infections which can be treated with the compounds of the invention include yellow fever virus, dengue fever virus, Japanese encephalitis virus and tick borne encephalitis virus.

Typical hepacivirus infections that can be treated with the compounds of the invention include hepatitis C virus.

Compounds of the present invention are especially active against hepatitis C. Typically, said flavivirus is therefore hepatitis C virus.

The compounds of the invention may be administered in a variety of dosage forms. Thus, they can be administered orally, for example as tablets, troches, lozenges, aqueous or oily suspensions, dispersible powders or granules. The compounds of the invention may also be administered parenterally, whether subcutaneously, intravenously, intramuscularly, intrasternally, transdermally or by infusion techniques. The compounds may also be administered as suppositories.

The compounds of the invention are typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacalogically inactive substances used in pharmaceutical formulations. Such pharmaceutical preparations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for injection or infusion may contain as carrier, for example, sterile water or preferably they may be in the form of sterile, aqueous, isotonic saline solutions.

Compounds of the present invention may be used in conjunction with known anti-viral agents. Preferred known anti-viral agents in this regard are interferon and ribavirin, and derivatives thereof, which are known for the treatment of hepatitis C (Clinical Microbiology Reviews, Jan, 2000, 67-82). The said medicament therefore typically further comprises interferon or a derivative thereof and/or ribavirin or a derivative thereof. Further, the present invention provides a pharmaceutical composition comprising:

(a) a quinazoline derivative of the formula (Ia) and (Ib), as defined above, or a pharmaceutically acceptable salt thereof;
(b) interferon or a derivative thereof and/or ribavirin or a derivative thereof; and
(c) a pharmaceutically acceptable carrier or diluent.

Also provided is a product comprising:

(a) a quinazoline derivative of the formula (Ia) and (Ib), as defined above, or a pharmaceutically acceptable salt thereof; and
(b) interferon or a derivative thereof and/or ribavirin or a derivative thereof, for separate, simultaneous or sequential use in the treatment of the human or animal body.

A preferred interferon derivative is PEG-interferon. A preferred ribavirin derivative is viramidine.

Further, the compounds of the invention are found to interact synergistically with interferon. Typically, therefore, component (b) of the above pharmaceutical composition or product is interferon, more typically a type I interferon, preferably an interferon $\alpha$.

A preferred interferon is an interferon $\alpha$2b, which is typically pegylated, for example PEG-Intron (Schering Plough Corp) or a protein fusion product such as Albuferon (Human Genome Sciences). The interferon $\alpha$2b may also be formulated for controlled release.

Another preferred interferon is interferon is an interferon $\alpha$2a. Preferably, the interferon $\alpha$2a is pegylated, for example Pegasys (Roche) and Roferon A (Roche).

Another preferred interferon is interferon $\alpha$8 (Riotech).

Another preferred interferon is interferon alfacon-1 (Intermune), preferably pegylated interferon alfacon-1

In another embodiment, the interferon is interferon $\beta$, preferably interferon $\beta$-1a. (Serono SA).

In another embodiment the interferon is interferon gamma (Intarcia).

Preferably, the interferon is contained in a formulation or device which allows the interferon to be released in a controlled manner, Examples are the DUROS implant technologies developed by ALZA Corp and the SABER drug delivery technology developed by Durect Corp.

The present invention also provides the use of a quinazoline derivative of the formula (Ia) and (Ib), as defined above, or a pharmaceutically acceptable salt thereof, in the manufacture of a medicament for use in treating or preventing an HCV infection by co-administration with a said interferon or interferon derivative. Also provided is the use of a said interferon or interferon derivative, in the manufacture of a medicament for use in treating or preventing an HCV infection, by co-administration with a quinazoline derivative of the formula (Ia) and (Ib), as defined above, or a pharmaceutically acceptable salt thereof.

A therapeutically effective amount of a compound of the invention and, when desired, a said interferon or interferon derivative is administered to a patient. A typical dose is from about 0.01 to 100 mg per kg of body Weight, according to the activity of the specific compound, the age, weight and conditions of the subject to be treated, the type and severity of the disease and the frequency and route of administration. Preferably, daily dosage levels are from 0.05 to 16 mg per kg of body weight, more preferably, from 0.05 to 1.25 mg per kg of body weight.

The compounds of the present invention may also be used with other antiviral agents. Preferred antiviral agents in this regard are cyclosporins, interleukin 2, interleukin 6, interleukin 12, interfering RNA or antisense RNA, all of which

are known for the treatment of Hepatitis C. Compounds of the present invention may also be used in conjunction with other inhibitors of the HCV nucleic acid sequences or HCV protein targets. Preferred HCV protein targets include HCV serine protease as illustrated by VX-950 from Vertex and SCH-503034 from Scering Plough both protease inhibitors, HCV polymerase as illustrated by HCV-796 from Wyeth, HCV helicase, HCV NS4B and HCV NS5A. Further, the compounds of the present invention may also be used in conjunction with inhibitors of HCV entry into the cell or inhibitor of interaction of HCV with host cell proteins.

The following Examples illustrate the invention. They do not however, limit the invention in any way. In this regard, it is important to understand that the particular assay used in the Examples section is designed only to provide an indication of anti-viral activity. There are many assays available to determine such activity, and a negative result in any one particular assay is therefore not determinative.

## EXAMPLES

Materials and Methods:

All temperatures are in °C. Thin layer chromatography (TLC) was carried out on Si 60G coated plastic plates with $UV_{254}$ indicator (Polygram). All NMR spectra were obtained at 250MHz in $d^6$-DMSO unless stated otherwise, chemical shifts expressed as ppm, apparent coupling constants (J) given for obvious multiplicities.

"Concentrated" implies solvents were removed *in vacuo.* All solids were dried at 40 °C.

LC-MS CONDITIONS

Samples were run on a MicroMass ZMD, using electrospray with simultaneous positive - negative ion detection.
Column : Synergi Hydro-RP, 30 x 4.6mm I.D, 4μm.
Gradient: 95:5 to 5:95 v/v $H_2O/CH_3CN$ + 0.05% Formic Acid over 4.0 min, hold 3 min, return to 95:5 v/v $H_2O/CH_3CN$ + 0.05% Formic Acid over 0.2 min and hold at 95:5 v/v $H_2O/CH_3CN$ + 0.05% Formic Acid over 3 min.
Detection : PDA 250 - 340 nm.
Flow rate : 1.5 ml/min.
All retention times (rt) are expressed in minutes.

Experimental:

Intermediate 1: 2-amino-5-iodobenzonitrile

Prepared by the method of A. Rosowsky & H. Chen, J. Org. Chem. 2001, 66, 7522-7526.
$^1H$ NMR ($CDCl_3$) δ 7.64 (1H, s), 7.55 (1H, dd, J 8.5, 2.5Hz), 6.53 (1H, d, J 8.5Hz), 4.66 (2H, br s); LC-MS rt 2.42 m/z 243 ES-.

Intermediate 2: N'-(2-Cyano-4-iodo-phenyl)-N,N-dimethyl-formamidine

A solution of 2-amino-5-iodobenzonitrile (50g, 0.2mol) in DMF-DMA (2.5 eq, 6 ml) was heated to 120 ° for 2 h. The excess DMF-DMA was removed by concentration to leave the title compound as a viscous brown oil (61 g, quant). Solidifies on standing at 4 °.
$^1H$ NMR ($CDCl_3$) δ 7.79 (1H, d, J 1.9Hz), 7.65 (1H, dd, J 1.9, 8.5Hz), 7.57 (1H, s), 6.70 (1H, d, J 8.2Hz), 3.08 (6H, s); LC-MS rt 2.1 M/z 300 ES+

Intermediate 3: 2-amino-5-bromobenzonitrile

A solution of 2-aminobenzonitrile (11.8g, 0.1 mol) in AcOH (120 ml) was treated with ammonium bromide (10.3 g, 0.105 mol) and hydrogen peroxide (10.2 ml, 35 % in water, 0.105 mol). This mixture was stirred at room temperature for ca. 24 hours until LCMS analysis showed the reaction was complete. The mixture was concentrated to remove the AcOH, prior to stirring the residue with 30 % aqueous NaOH solution until basic. The resulting solid was removed by filtration and washed with water before drying. This solid was then dissolved in an excess of DCM. The solution was concentrated until precipitation started and then allowed to stand until crystallisation was complete. The resulting solid was removed by filtration and washed with a little DCM. This gave the desired compound as an off white crystalline solid (19.2 g, 97%).
$^1H$ NMR δ 7.61 (1H, d, J 2.5Hz), 7.43 (1H, dd, J 9, 2.5Hz), 6.75 (1H, d, J 9Hz), 6.28 (2H, br s); LC-MS rt 2.24

Intermediate 4: N'-(4-bromo-2-cyano-phenyl)-N,N-dimethyl-formamidine

A solution of 2-amino-5-bromobenzonitrile (10.25 g, 52 mmol) in DMF-DMA (20 ml) was heated to reflux for 1 hour. The mixture was cooled and concentrated to dryness before triturating with t-butyl-methyl-ether (TBME) (10 ml). Petrol (30 ml) was then added and the solid scratched, and allowed to stand for 1 hour. The solid product was filtered off and washed with TBME/petrol (1:2) to afford a crystalline solid (11.95 g, 91.2 %).
$^1H$ NMR ($CDCl_3$) δ 7.55 (1H, d, J 2Hz), 7.51 (1H, s), 7.41 (1H, dd, J 2, 9Hz), 6.75 (1H, d, J 9Hz), 3.01 (6H, s); LC-MS rt 1.95 m/z 252/254 ES-.

Intermediate 5: 2-Amino-5-(4,4,5,5-tetramethyl-[1,3,2]dioxaborolan-2-yl)-benzonitrile

A mixture of Pd $Cl_2$ (dppf) (3.35 g), potassium acetate (12.07 g) and bis(pinacolato)boron (12.48 g) in dry DMF (80 ml) was treated with Intermediate 1 (10 g) and heated to 80 ° for 4 h. The cooled mixture was partitioned between water (400 ml) and DCM (400 ml). The aqueous phase was further extracted with DCM (2 x 100 ml) and the combined organic phases dried and concentrated. The residue was purified by chromatography on silica gel (90 g, MPLC) with 10-30 % EtOAc in petrol as eluant. Concentration of fractions containing product and trituration with further petrol gave the desired product as a white solid (6.91 g, 69 %)
$^1H$ NMR($CDCl_3$) δ 7.87 (1H, s), 7.72 (2H, d, J 8.21), 6.7 (1H, d, J 8.2Hz), 4.57 (2H, br s), 1.31 (12H, s); LC-MS rt 2.84 m/z 244 ES+.

Intermediate 6: N'-[2-Cyano-4,4,5,5-tetramethyl-[1,3,2]dioxaborolan-2-yl)-phenyl]-N,N-dimethyl-formamidine

A suspension of Intermediate 5 (750mg) in DMF-DMA (1ml) was heated to 100 ° under $N_2$ for 30 mins then cooled to rt. The solvent was removed and the residue purified by SPE on silica gel (5 g) with 10% EtOAc/petrol as eluant This gave the title compound as a clear oil which crystallised on standing (915 mg, 100 %).
$^1H$ NMR($CDCl_3$) 7.98 (1H, s), 7.817 (1H, d, J 8.2Hz), 7.62 (1H, s), 6.92 (1H, d, J 7.6Hz), 3.1 (3H, s), 3.07 (3H, s), 1.33 (12H, s); LC-MS rt 2.73m/z 300 ES+

Intermediate 7: 4-(4-Iodo-phenoxymethyl)-pyridine

A mixture of 4-iodophenol (1 g) $K_2CO_3$ (powdered, 1.88 g) and 4-picolyl chloride (822 mg) in acetone (15 ml) was heated at reflux for 16 h. A further portion of 4-picolyl chloride (411mg) added and heating continued for 6 h. Cooled, filtered and the filtrate adsorbed onto silica and purified by MPLC (35 g Si, 10-50 % EtOAc in petrol gradient elution over 30 min). This gave, on concentration, a white solid (710 mg, 50 %).
$^1H$ NMR($CDCl_3$) δ 8.64 (2H, d, J 5.69Hz), 7.6 (2H, d, J 8.85Hz), 7.35 (2H, d, J 5.69Hz), 6.76 (2H, d, J 8.85Hz), 5.08 (2H, s).

Intermediate 8: 4-Bromo-1,2-diethoxy-benzene

To a well stirred mixture of diethoxybenzene (500 mg) and ammonium bromide (323 mg, 1.1 eq) in MeCN ( 20 ml) was added oxone (2.03 g, 1.1 eq). This suspension was stirred at rt for 4 h then the suspension filtered and the filtrate concentrated to give the title compound (723 mg,> 90 %) which was used without further purification.
$^1H$ NMR ($CDCl_3$) 6.98 (2H, m), 6.73 (1H, d, J 8.85Hz), 4.05 (4H, m), 1.44 (6H, m); LC-MS rt 2.48 m/z 279 ES+.

Intermediate 9: 4-Bromo-1-(2-bromoethoxy)-2-fluorobenzene

To a stirred solution of 4-bromo-2-fluorophenol (3.5g, 18.3mmol, 1eq), 2-bromoethanol (3.44g, 27.5mmol, 1.5eq) and triphenylphosphine (7.21g, 27.5mmol, 1.5eq) in THF (50mL) at 0°C, under nitrogen, was added DEAD (4.78g, 27.5mmol, 1.5eq) drop-wise *via* syringe. The reaction was then allowed to warm to room temperature. After 2h the reaction was concentrated to dryness *in vacuo* and the off-white residue placed on top of a short pad of silica and washed with 9:1 petroleum spirit:EtOAc (3 x 50 mL). The filtrate was concentrated to give the title compound as a clear oil (5.28g, 97%).
$^1H$-NMR (DMSO-$d_6$), 3.57 (t, 2H), 4.26 (t, 2H), 6.80 (m,1H), 7.12 (m, 1H), 7.19 (m, 1H). LC-MS rt 2.90 m/z no ion.

Intermediate 10: *N*-[2-(4-bromo-2-fluorophenoxy)ethyl]-*N',N',N'*-trimethylethane-1,2-diamine

A mixture of Intermediate 9 (3g, 10mmol, 1eq), *N,N,N'*-trimethylethylenediamine (1.54g, 15mmol, 1.5eq) and potassium carbonate (2.09g, 15mmol), 1.5eq) in acetone was heated at reflux. After 16h the reaction was allowed to cool to room temperature and then filtered. The filtrate was concentrated *in vacuo* to give a pale orange syrup. The syrup

was purified by flash column chromatography, eluting initially with 100% $CH_2Cl_2$ and then 100:8:1 $CH_2Cl_2$:EtOH:$NH_3$. The tile compound was isolated as a pale orange syrup (2.24g, 70%). $R_f$ = 0.12 (100:8:1 $CH_2Cl_2$:EtOH:$NH_3$).
$^1$H-NMR (DMSO-$d_6$) 2.41 (s, 3H), 2.57 (s, 6H), 2.82 (m, 4H), 2.91 (t, 2H), 4.14 (t, 2H), 6.89 (m, 1H), 7.21 (m, 1H), 7.27 (m, 1H). LC-MS rt 1.79 m/z 320 ES+.

Intermediate 11: *N'*-(3-cyano-4'-{2-[(2-dimethylaminoethyl)methylamino]ethoxy}-3'-fluorobiphenyl-4-yl)-*N,N*-dimethylformamidine

A mixture of Intermediate 10 (950mg, 2.98mmol), 1eq), bis(pinacolato)diboron (1.51g, 5.95mmol, 2eq), potassium acetate (1.02g, 10.43mmol), 3.5eq) and $PdCl_2(dppf)_2CH_2Cl_2$ (245mg, 0.30mool, 0.1eq) in DMF (10mL) was heated at 80°C, under nitrogen. After 16h the reaction was allowed to cool to room temperature and concentrated *in vacuo* to give a brown residue. The residue was extracted with EtOAc (3 x 20mL) and the extracts were combined and concentrated *in vacuo* to dryness to give a brown oil (1.09g). A mixture of the oil (1.09g), *N'*-(2-cyano-4-iodophenyl)-*N,N*-dimethylformamide (811mg, 2.71mmol) and tetrakis(triphenylphosphine)palladium (162mg, 0.14mmol) in DME (10mL) and a saturated aqueous solution of $Na_2CO_3$ (5mL) was heated at reflux. After 20h the reaction was allowed to cool to room temperature and then concentrated *in vacuo* to give a brown residue. The residue was purified by flash column chromatography eluting with 800:8:1, 200:8:1 and 100:8:1 $CH_2Cl_2$:EtOH:$NH_3$. The title compound was isolated as a pale brown oil (273mg, 22%). $R_f$= 0.08 (100:8:1 $CH_2Cl_2$:EtOH:$NH_3$).
$^1$H-NMR (DMSO-$d_6$) 2.20 (s, 6H), 2.33 (s, 3H), 2.41 (t, 2H), 2.57 (t, 2H), 2.81 (t, 2H), 3.03 (s, 3H), 3.05 (s, 3H), 4.11 (t, 2H), 6.95 (m, 2H), 7.17 (m, 2H), 7.58 (m, 2H), 7.61 (m, 1H). LC-MS rt 1.86 m/z 412 ES+.

Intermediate 12: 2-Methoxy-5-bromophenol

Prepared by the method of Meyers and Snyder, J. Org. Chem, 1993, 58,1, 42.
$^1$H NMR ($CDCl_3$) 7.00 (1H, d, J 2.25Hz), 6.90 (1H, dd, J 8.5, 2.25Hz), 6.66 (1H, d, J 8.75Hz), 5.57 (1H, s), 3.81 (3H, s)

Intermediate 13: 5-Bromo-2-fluorophenol

Prepared by the method of M Elliott, N Janes & B Khambay, GB2187731.
$^1$H NMR ($CDCl_3$) 7.08 (1H, m), 6.89 (2H, m), 5.14 (1H, s)

Intermediate 14: 4-Bromo-2-isopropoxy-1-methoxy-benzene

To a solution of Intermediate 12 (279mg, 1.37mmol) in DMF (5ml) was added potassium carbonate (945mg, 6.85mmol) and 2-iodopropane (684μl, 6.85mmol). The reaction mixture was stirred at 90°C over 4hrs. Analysis by LC-MS showed 20% starting phenol remaining so another portion of 2-iodopropane (684μl, 6.85mmol) was added and the mixture stirred at 90°C overnight.

LC-MS analysis showed consumption of all starting phenol so the reaction mixture was filtered and the filtrate was diluted with water (50ml) and extracted into ethyl acetate three times. The combined extracts were dried over magnesium sulphate, filtered and evaporated under reduced pressure yielding a dark brown oil (294mg, 87%).
$^1$H NMR ($D^6$-DMSO) 6.93 (2H, m), 6.68 (1H, d, J 8.75Hz), 4.43 (1H, m), 3.75 (3H, s), 1.31 (6H, d, J 6Hz); LC-MS rt 2.80.

Intermediate 15: 4-Bromo-1-fluoro-2-isopropoxy-benzene

To a solution of Intermediate 13 (262mg, 1.37mmol) in DMF (5ml) was added potassium carbonate (945mg, 6.85mmol) and 2-iodopropane (684μl, 6.85mmol). The reaction mixture was stirred at 90°C over 4hrs. Analysis by LCMS showed 20% starting phenol remaining so another portion of 2-iodopropane (684μl, 6.85mmol) was added and the mixture stirred at 90°C overnight.
LCMS analysis showed consumption of all starting phenol so the reaction mixture was filtered and the filtrate was diluted with water (50ml) and extracted into ethyl acetate three times. The combined extracts were dried over magnesium sulphate, filtered and evaporated under reduced pressure yielding a dark brown oil (274mg, 86%).
$^1$H NMR ($D^6$-DMSO) 7.00 (1H, dd, J 2.25Hz), 6.92 (2H, m), 4.45 (1H, m), 1.30 (6H, d, J 6.25Hz)
LC-MS rt 2.93

Intermediate 16: 4-Amino-3'-isopropoxy-4'-methoxy-biphenyl-3-carbonitrile

To a solution of Intermediate 14 (294mg, 1.2mmol) in dimethoxyethane (4ml) was added Intermediate 5 (439mg, 1.8mmol) followed by sat. aq. Sodium carbonate (2ml) and tetrakis(triphenylphosphine)palladium (0) (139mg, 0.12mmol).

This reaction mixture was stirred at 80°C overnight. LCMS analysis showed consumption of starting material so reaction mixture was diluted with water and extracted into dichloromethane three times. The combined extracts were dried over magnesium sulphate, filtered and evaporated under reduced pressure. Column chromatography (gradient 0 - 50% EtOAc in petroleum ether) furnished the desired product as a yellow solid (137mg, 40%).
$^1$H NMR ($CDCl_3$) 7.47 (2H, m), 6.94 (2H, m), 6.87 (1H, d, J 9Hz), 6.75 (1H, d, J 8.5Hz), 4.55 (1H, quin, J 6.25Hz), 4.37 (2H, s), 3.81 (3H, s), 1.34 (6H, d, J 6.25 Hz); LC-MS rt 2.70

Intermediate 17: 4-Amino-4'-fluoro-3'-isopropoxy-biphenyl-3-carbonitrile

To a solution of Intermediate 15 (274mg, 1.18mmol) in dimethoxyethane (4ml) was added Intermediate 5 (439mg, 1.8mmol) followed by sat. aq. Sodium carbonate (2ml) and tetrakis(triphenylphosphine)palladium (0) (139mg, 0.12mmol). This reaction mixture was stirred at 80°C overnight.
LCMS analysis showed consumption of starting material so reaction mixture was diluted with water and extracted into dichloromethane three times. The combined extracts were dried over magnesium sulphate, filtered and evaporated under reduced pressure. Column chromatography (gradient 0 - 50% EtOAc in petroleum ether) furnished the desired product as a yellow solid (196mg, 60%).
$^1$H NMR ($CDCl_3$) 7.41 (2H, m), 6.97 (3H, m), 6.72 (2H, d, J 8Hz), 4.53 (1H, quin, J 6Hz), 4.40 (2H, s), 1.33 (6H, d, J 6Hz); LC-MS rt 2.86

Intermediate 18: N'-(3-Cyano-3'-isopropoxy-4'-methoxy-biphenyl-4-yl)-N,N-dimethyl-formamidine

A solution of Intermediate 16 (137mg, 0.41mmol) in DMF-DMA (2ml) was stirred at 80°C overnight. LCMS analysis shows consumption of starting material. Reaction mixture evaporated under reduced pressure and then redissolved in toluene and evaporated under reduced pressure again. Column chromatography (gradient 20 - 40% EtOAc in petroleum ether) furnished the desired compound as a dark yellow oil which solidified on standing (120mg, 87%)
$^1$H NMR ($CDCl_3$) 7.63 (1H, d, J 2Hz), 7.57 (1H, s), 7.54 (1H, dd, J 8.5Hz, 2.25Hz), 7.00 (2H, s), 6.91 (2H, m), 4.54 (1H, quin, J 6Hz), 3.82 (1H, s), 3.05 (6H, d, J 6.5Hz), 1.35 (6H, d, J 6Hz); LC-MS rt 2.40 m/z 338 ES+

Intermediate 19: N'-(3-Cyano-4'-fluoro-3'-isopropoxy-biphenyl-4-yl)-N,N-dimethyl-formamidine

A solution of Intermediate 17 (196mg, 0.60mmol) in DMF-DMA (2ml) was stirred at 80°C overnight. LCMS analysis shows consumption of starting material. Reaction mixture evaporated under reduced pressure and then redissolved in toluene and evaporated under reduced pressure again. Column chromatography (gradient 20 - 40% EtOAc in petroleum ether) furnished the desired compound as a dark yellow oil which solidified on standing (190mg, 97%).
$^1$H NMR ($CDCl_3$) 7.57 (2H, m), 7.49 (1H, dd, J 8.5Hz, 2.25Hz), 6.99 (4H, m), 4.53 (1H, quin, J 6Hz), 3.02 (6H, d, J 5.75Hz), 1.32 (6H, d, J 6Hz); LC-MS rt 2.71 m/z 326 ES+

Intermediate 20: 1-(3-Methyl-4-nitro-phenyl)-1H-[1,2,4]triazine

To a 50ml round bottom flask was added the 4-fluoro-2-methyl-1-nitrobenzene (0.5g, 3.22mmol), $Na_2CO_3$ (0.36g, 3.38mmol) and 1,2,4-triazole (0.22g, 3.22 mmol) in DMF (DRY 10ml). The mixture was stirred at 125°C for 24 hr under nitrogen. The DMF was evaporated to dryness and the crude product was put on a silica column and eluted with 2.5% MeOH : DCM. Isolated 0.47g (72%) of a white solid.
$^1$H n.m.r ($D_6$-DMSO) 9.52 (1H, s), 8.39 (1H, s), 8.27 (1H, d, J = 8.85Hz), 8.13 (1H, d, J = 1.89Hz), 8.02 (1H, dd, J = 8.85Hz, 2.53Hz), 2.68 (3H, s); LC-MS rt 2.26 m/z 205 ES+

Intermediate 21: 2-Methyl-4-[1,2,4]triazol-1-yl-phenylamine

To a 50ml round bottom flask was added Intermediate 20(0.47g, 2.3mmol) and EtOH (10ml). To this stirred mixture at room temperature was added $SnCl_2.2H_20$ (2.5g, 11.5mmol). The mixture was then heated at 80°C for 4 hr. The mixture was allowed to cool and the pH of the solution was taken to 8 via addition of 2N NaOH. The mixture was filtered and concentrated to dryness then partitioned between DCM:$H_20$ (50ml:25ml). The aqueous layer was separated and washed again with DCM (25ml). The DCM fractions were combined and put through a hydrophobic frit to remove water. The filtrate was concentrated to dryness to afford a brown solid 0.3g (75%).
$^1$H n.m.r ($D_6$-DMSO) 9.02 (1H, s), 8.15 (1H, s), 7.43 (1H, d, J = 1.89Hz), 7.37 (1H, dd, J = 8.20Hz, 2.52Hz), 6.76 (1H, d, J = 8.85Hz), 5.20 (2H, s), 2.18 (3H, s); LC-MS rt 1.33 m/z 175 ES+

Intermediate 22: N'-[3-Cyano-3'-fluoro-4'-(2-methoxy-ethoxy)-biphenyl-4-yl]-N,N-dimethyl-formamidine

*Step 1: 4-Bromo-2-fluoro-1-(2-methoxy-ethoxy)-benzene*

A mixture of 4-fluorophenol (9.8ml), powdered potassium carbonate (2eq, 24g) and bromoethyl methyl ether (1.1eq, 20.16ml) in acetone (60ml) was heated to reflux overnight. The cooled reaction mixture was diluted with water and extracted into EtOAc. The combined organic phases were washed with aqueous sodium carbonate, dried ($Na_2SO_4$ and concentrated to give the product as a mobile oil (22g, quantitative).
$^1$H n.m.mr. ($CDCl_3$) δ 7.14 (2H, m), 6.84 (1H, t, J 8.85Hz), 4.09 (2H, m), 3.69 (2H, m), 3.38 (3H, s); LC-MS rt 2.67 m/z no ion

*Step 2: 3-Fluoro-4-(2-methoxy-ethoxy)-phenylboronic acid*

To a solution of 4-bromo-2-fluoro-1-(2-methoxy-ethoxy)-benzene (4.98g) in THF (40ml) was added a small crystal of iodine and then Mg (730mg, 1.5eq) portion-wise. After addition, the mixture was heated to reflux for 4h. The grey mixture was cooled to -78° trimethylborate (1.2eq), 2.25ml) added and allowed to warm overnight. 1N HCl (aq, 60ml) was added and stirred for 30 min before being extracted into ether (2x50ml). The combined organic phases were dried and concentrated and the resulting solid triturated with ether / petrol, isolated by filtration and dried to give the *title compound* as a cream solid (3.122g, 73%)
$^1$H NMR ($CDCl_3$) δ 8.04 (2H, br s), 7.53 (2H, m), 7.12.(1H, t, J 8.85Hz), 4.17 (2H, m), 3.67 (2H, m), 3.3 (3H, s); LC-MS rt 1.97 m/z 213 ES-

*Step 3: N'-[3-Cyano-3'-fluoro-4'-(2-methoxy-ethoxy)-biphenyl-4-yl]-N,N-dimethyl-formamidine*

A mixture of 3-fluoro-4-(2-methoxyethoxy)-phenylboronic acid (1.83g, 1.2eq), intermediate 2 (2.32g), potassium carbonate (1.29g, 1.2eq) in DMF / $H_2O$ (3:1, 40ml) wass treated with dichloro(bis benzonitrile) palladium (II) (1%, 30mg) and stirred at ambient under $N_2$ for 4h. The mixture was diluted with water (100ml) and filtered then extracted with EtOAc (2x50ml) and these organic extracts added to the filter cake, dried and concentrated. The residue was dissolved in DCM, loaded onto a short column of silica and eluted portion-wise under suction with DCM/EtOH/$NH_3$ 400-200:8:1. On concentration, this gave a brown oil which was triturated with DCM/ ether/ petrol and on crystallization diluted with further petrol (60ml). The title compound was isolated by filtration as a cream solid (1.665g, 63%)
$^1$H NMR ($CDCl_3$) δ 7.74 (1H, d, J 1.9Hz), 7.7(1H, s), 7.60 (1H, dd, J 8.85, 1.9Hz), 7.29 (3H, m), 7.1.(1H, d, J 8.2Hz), 7.05 (1H, d, J8.85Hz), 4.28 (2h, m), 3.84 (2H, m), 3.52 (3H, s), 3.16 (3H, s), 3.14 (3H, s); LC-MS rt 2.32 m/z 342 ES+

Intermediate 23: N'-(3-Cyano-3',4'-dimethoxy-biphenyl-4-yl)-N,N-dimethyl-formamidine

Intermediate 4 (2.52g, 10 mmol) and 3,4-dimethoxyphenyl boronic acid (1.9g, 1.2eq) in iPrOH (30ml) were treated with 2N aqueous sodium carbonate (10ml) and tetrakis-(triphenylphosphine) palladium (0) (5mg, 0.04mol%) and heated with stirring at 60°. After 2 hours the reaction was shown to be ca. 80% complete by LCMS. The reaction mixture was then cooled and diluted with water (30ml). The resulting solid was filtered off, washed with further water (2x20ml) before drying by suction. The solid was re-slurried twice in ether (2x5ml) sucked dry, and finally dried to give the biphenyl intermediate as an off white solid (2.24g, 72%).
$^1$H NMR ($D^6$-DMSO) δ 7.99 (1H, s) 7.91 (1H, d, J 1.9Hz), 7.79 (1H, dd, J 8.2, 1.9Hz), 7.2 (3H,m), 6.99 (1H, d, J 8.2Hz), 3.84 (3H, s), 3.78 (3H, s), 3.08 (3H, s) 3.01 (3H, s); LC-MS rt 2.31 m/z 309 ES-

Intermediate 24: 1-(4-Amino-phenyl)-5-methyl-1H-[1,2,4]triazole-3-carboxylic acid

A solution of 5-methyl-1-(4-nitrophenyl)-1H-1,2,3-triazole-3-carboxylic acid (100mg, 0.403 mM) in methanol (8ml) was injected at a 1ml/min rate into an hydrogenator "the H-Cube" that combines endogenous hydrogen generation with a disposable cartridge system (Pd/C), temperature was set up to 25°C at atmospheric pressure.
The solution obtained was concentrated to give a white solid (82mg, 93%).
$^1$H NMR ($D^6$-DMSO) δ 8.72 (1H, broad s), 8.59 (1H, broad s), 7.42 (2H, d, J 8Hz), 7.23 (1H, d, J 8Hz), 6.99 (2H, d, J 8Hz), 6.70 (1H, d, J 8Hz), 2.48 (3H, s), 2.45 (3H, s).

Intermediate 25: 1-(4-Amino-phenyl)-5-methyl-1H-[1,2,4]triazole-3-carboxylic acid dimethylamide.

*Step1: 5-Methyl-1-(4-nitrophenyl)-1H-[1,2,4]triozazole-3-carboxylic acid dimethiylamide.*

To a solution of 5-methyl-1(4-nitrophenyl)-1H-1,2-4-triazole-3-carboxylic acid (Key organics 400mg, 1.61mM)

and Hunig's base (667ul, 3.86mM) in DCM:DMF (6ml:1ml) at -10°C was added dropwise isobutylchloroformate (250ul, 1.93mM). The reaction was stirred 30min. at -10°C then slowly a 2M solution of dimethylamine (965ul, 1.93mM) was added. The mixture was allowed to warm-up to room temperature and stirred for another hour. The crude mixture was washed with sat. $NaHCO_3$ extracted and dried over $MgSO_4$. After evaporation the yellow solid obtained was used directly in the next reaction without further purification (412mg, 93%). LC-MS rt 2.09 m/z 275 ES+
$^1$H NMR (D$^6$-DMSO) δ 8.42 (2H, d, J 7Hz), 7.95 (2H, d, J 7Hz), 3.13 (3H, s), 3.03 (3H, s), 2.62 (3H, s).

*Step 2: 1-(4-Amino-phenyl)-5-methyl-1H-[1,2,4]triazole-3-carboxylic acid dimethylamide.*

A solution of 5-methyl-1-(4-nitrophenyl)-1H-1,2,3-triazole-3-carboxylic acid methylamide from step1(100mg, 0.36mM) in methanol (8ml) was injected at a 1ml/min rate into an hydrogenator "the H-Cube" that combines endogenous hydrogen generation with a disposable cartridge system (Pd/C), temperature was set up to 25°C at atmospheric pressure. The solution obtained was concentrated to give a white solid (87mg, 98%). LC-MS: rt 1.54 m/z 245 ES$^+$
$^1$H NMR (D$^6$-DMSO) δ 8.71 (1H, broad s), 8.54 (2H, d, J 7Hz), 7.12 (2H, d, J 7Hz), 3.13 (3H, s) 3.03 (3H, s), 2.62 (3H, s).

<u>Example 1: 6-Iodo-quinazolin-4-yl-(4-[1,2,4]triazol-1-yl-phenyl)-amine</u>

4-Chloro-6-iodoquinazoline (WO9609294 A1, 150 mg) was treated with 4-triazolyl aniline (28 mg, 1 eq) in MeCN and refluxed for 6 h. Cooled overnight and filtered to give the title compound (70 mg).
$^1$H NMR δ 11.75 (1H, br s), 9.4 (2H; s), 9.04 (1H, s), 8.43 (1H, d, J 8.85Hz), 8.34 (1H, s), 8.0(4H, m), 7.82 (1H, d, J 8.85Hz); LC-MS rt 2.32 m/z 415 ES+.

<u>Example 2: (6-tert-Butyl-quinazolin-4-yl)-(4-[1,2,4]triazol-1-yl-phenyl)-amine</u>

A solution of 2-amino-5-tert-butylbenzoic acid (commercial sources, 500 mg) and formamidine acetate (404 mg) in EtOH (5 ml) was refluxed for 18 h. The cooled mixture was filtered and the precipitate washed with ice-cold EtOH and dried to give the hydroxy quinazoline (394 mg) which was added to thionyl chloride (10 ml) and DMF (cat.) and heated to reflux overnight. The cooled mixture was diluted with EtOAc and poured onto sat sodium bicarbonate (aq). The organic phase was separated, dried and concentrated to a brown solid (327 mg), of which a portion (105 mg) was treated immediately with 4-triazolylaniline (125 mg) in MeCN (4 ml) at reflux overnight. The cooled mixture was partitioned between DCM and sodium bicarbonate and the organic phase concentrated. Purification by chromatography with DCM: EtOH:$NH_3$ (200:8:1) as eluant gave the desired compound.
$^1$H NMR δ 10.16 (1H, s), 9.48 (1H, s), 8.77 (1H, s), 8.62 (1H, s), 8.41 (1H, d), 8.2 (4H, m), 7.95 (1H, d), 1.64 (9H, s); LC-MS rt 2.18 m/z 343 ES-.

<u>Example 3: [7-(3-Chloro-propoxy)-6-methoxy-quinazolin-4-yl]-(4-[1,2,4]triazol-1-yl phenyl)amine</u>

N'-[5-(3-Chloro-propoxy)-2-cyano-4-methyoxy-phenyl]-N,N-dimethyl-formamidine (WO0305549 580 mgs), 4-(1,2,4-triazolyl)aniline (314 mgs) and AcOH (4 mL) were heated to 90° for 1 hr before cooling. The oil was concentrated and the resultant slurry dissolved in MeOH, sonicated and the resultant white powder filtered and dried to produce the title compound (765 mg).
$^1$H NMR δ 9.65 (s, 1H), 9.25 (s, 1H), 8.5 (s, 1H), 8.23 (s, 1H), 8.02 (d, 2H, *J* 9Hz), 7.89 (s, 1H), 7.87 (d, 2H, *J*=9), 7.24 (s, 1H), 4.28 (t, 2H, *J*=6), 3.95 (s, 3H), 3.83 (t, 2H, *J* 6Hz), 2.27 (q, 2H, *J* 6Hz); LC-MS rt 2.13 m/z 411 ES+.

<u>Example 4: [6-Methoxy-7-(3-morpholin-4-yl-propoxy)-quinazolin-4-yl]-(4-[1,2,4]triazol-1-yl-phenyl)-amine</u>

Example 3 (118.6 mgs, 0.289 mmol), morpholine (120 μL) and dimethylacetamide (1.1 mL) were heated to 90° for 12 hr before cooling, concentrating and purifying the resultant oil by column chromatography with 200:8:1, DCM: MeOH:$NH_3$ as eluant. The oil was recrystallised from MeCN producing the title compound as colourless crystals, (126 mg).
$^1$H NMR δ 9.55 (s, 1H), 8.57 (s, 1H), 8.02 (s, 1H), 7.80 (d, 2H, *J*8.9) 7.35 (s, 1H), 7.19 (d, 2H, *J*=8.9), 4.38 (t, 2H, *J* 6Hz), 4.15 (s, 3H), 4-3.92 (m, 4H), 3.83-3.75 (m, 4H), 3.36-3.28 (m, 4H); LC-MS rt 1:78 m/z 462 ES+

<u>Example 5: [6-(3,4-Difluoro-phenul)-quinazolin-4-yl]-(4-[1,2,4]triazol-1-yl-phenyl)-amine</u>

*Step 1: N'-(3-Cyano-3',4'-difluoro-biphenyl-4-yl)-N,N-dimethyl-formamidine*

A mixture of 3,4-difluorophenyl boronic acid (Lancaster, 396 mg, 1.5 eq), Intermediate 2 (500 mg) and tetrakis (triphenylphosphine)palladium (0) (5%, 96 mg) was heated in DME / 2N sodium carbonate (aq, 2:1,13.5ml) at reflux for

16 h. The mixture was concentrated, the residue washed with water and ether and dried to give a light brown solid (386 mg, 81 %).
$^1$H NMR ($CDCl_3$) δ 7.57 (1H, d, J = 2Hz), 7.54 (1H, s), 7.45 (1H, dd, J = 8.5, 2.25Hz), 7.13 (3H, m), 6.90 (1H, d, J = 8.5Hz), 3.0 (3H, s), 2.99 (3H, s); LC-MS rt 2.51; m/z 286 ES+.

*Step 2: [6-(3,4-Difluoro-phenyl)-quinazolin-4-yl]-(4-[1,2,4]triazol-1-yl-phenyl)-amine*

A mixture of formamidine from Step 1 (150 mg) and 4-triazolyl-aniline (88 mg, 1eq) in AcOH (2ml) was heated to 80 ° for 16 h. Cooled, concentrated and cautiously treated with sodium bicarbonate (aq). The resulting solid was isolated by filtration, washed with water then DCM:EtOH:$NH_3$ (20:8:1) added and refiltered. The filtrate was concentrated until a precipitate formed which was filtered, washed with ether and dried to give the title compound (82 mg, 34 %).
$^1$H NMR δ 10.0 (1H, s), 9.18 (1H, s), 8.75 (1H, s), 8.54 (1H, s), 8.12 (2H, m), 7.97 (1H, m), 7.92 (2H, m), 7.78 (3H, m), 7.66 (1H, m), 7.53 (1H, m); LC-MS rt 2.52 m/z 401 ES+.

Example 6: [6-(4-Chloro-3-fluoro-phenyl)-quinazolin-4-yl]-4-[1,2,4]triazol-1-yl-phenyl)-amine

*Step 1: N'-(4'-Chloro-3-cyano-3'-fluoro-biphenyl-4-yl)-N,N-dimethyl-formamidine*

A mixture of 4-chloro-3-fluorophenyl boronic acid (Combiblocks, 759 mg, 1.3 eq), Intermediate 2 (1 g) and tetrakis(triphenylphosphine) palladium (0) (5 %, 193 mg) was heated in DME / 2N sodium carbonate (aq, 2:1, 27 ml) at reflux for 16 h. The mixture was filtered, the filtrate concentrated, the residue washed with sat sodium bicarbonate, water and ether and dried to give the product (310 mg)
$^1$H NMR ($CDCl_3$) δ 7.58 (1H, d, J 2.0Hz), 7.53 (1H, s), 7.46 (1H, dd, J 8.5, 2.25Hz), 7.31 (1H, t, J 8.0Hz), 7.14 (1H, m), 7.12 (1H, m), 6.9 (1H, d, J 8.5Hz), 3.0 (3H, s), 2.98 (3H, s); LC-MS rt 2.70 ; m/z 302 ES+.

*Step 2: [6-(4-Chloro-3-fluoro-phenyl)-quinazolin-4-yl]-(4-[1,2,4]triazol-1-yl-phenyl)-amine.*

A mixture of formamidine from Step 1 (300 mg) and 4-triazolyl-aniline (167 mg, 1 eq) in AcOH (3 ml) was heated to 80 ° for 2 h. Cooled, and the resulting solid was isolated by filtration, washed with sodium bicarbonate, water and MeCN. The solid was purified by column chromatography on silica gel with DCM:EtOH:$NH_3$ (400:8:1 to 200:8:1) as eluant to give the title compound.
$^1$H NMR δ 10.36 (1H, br s), 9.29 (1H, s), 8.93 (1H, s), 8.63 (1H, s), 8.25 (2H, m), 8.03 (3H, m), 7.86 (2H, d, J 9.25Hz), 7.81 (3H, m); LC-MS rt 2.61 m/z 418 ES+.

Example 7: [6-(3-Fluoro-4-methoxy-phenyl)-quinazolin-4-yl]-(4-[1,2,4]triazol-1-yl-phenyl)-amine

*Step 1: N'-(3-Cyano-3'-fluoro-4'-methoxy-biphenyl-4-yl)-N,N-dimethyl-formamidine*

A mixture of 3-fluoro-4-methoxyphenyl boronic acid (Aldrich, 427 mg, 1.5 eq), Intermediate 2 (500 mg) and tetrakis(triphenylphosphine)palladium (0) (5 %, 96 mg) was heated in DME / 2N sodium carbonate (aq, 2:1,13.5 ml) at reflux for 16 h. The mixtures were concentrated, the residue washed with water and ether and dried to give a light brown solid (436 mg, 88 %).
$^1$H NMR ($CDCl_3$) δ 7.71 (1H, d, J = 2.25Hz), 7.67 (1H, s), 7.59 (1H, dd, J = 9.12 2.25Hz), 7.32 (1H, m), 7.27 (1H, m), 7.05 (2H, m), 3.96 (3H,s), 3.14 (3H, s), 3.12 (3H, s) ; LC-MS rt 2.33 m/z 298 ES+.

*Step 2*: *[6-(3-Fluoro-4-methoxy-phenzyl)-quinazolin-4-yl]-(4-[1,2,4]triazol-1-yl-phenyl)-amine*

A mixture of formamidine from Step 1 (200 mg) and 4-triazolyl-amline (112 mg, 1 eq) in AcOH (2 ml) was heated to 80 ° for 16 h. Cooled, concentrated and cautiously treated with sodium bicarbonate (aq). The resulting solid was isolated by filtration, washed with water then DCM:EtOH:$NH_3$ (20:8:1) added and refiltered. The filtrate was concentrated until a precipitate formed which was filtered, washed with ether and dried to give the title compound (129mg, 47%).
$^1$H NMR δ 10.06 (1H, s), 9.29 (1H, s), 8.81 (1H, s), 8.63 (1H, s), 8.23 (2H, m), 8.07 (2H, d, J 10.0Hz), 7.93 (1H, s), 7.88 (2H, m), 7.83 (1H, m); 7.73 (1H, d, J 7.50Hz), 7.35 (1H, t, J 8.75Hz), 3.92 (3H, s); LC-MS rt 2.42 m/z 413 ES+.

Example 8: [6-(4-Ethoxy-3-fluoro-phenyl)-quinazolin-4-yl]-(4-[1,2,4]triazol-1-yl-phenyl)-amine

*Step 1: N'-(3-Cyano-4'-ethoxy-3'-fluoro-biphenyl-4-yl)-N,N-dimethyl-formamidine*

A mixture of 4-ethoxy-3-fluorophenyl boronic acid (Combiblocks, 800 mg, 1.3 eq), Intermediate 2 (1g) and tetrakis(triphenylphosphine) palladium (0) (5 %, 193 mg) was heated in DME / 2N sodium carbonate (aq, 2:1, 27 ml) at reflux for 16h. The mixture was filtered, the filtrate concentrated, the residue washed with sat sodium bicarbonate, water and ether and dried to give the product (410mg).
$^1$H NMR δ 7.71 (1H, d, J = 2.0Hz), 7.67 (1H, s), 7.60 (1H, dd, J = 8.5 2.25Hz), 7.31 (1H, m), 7.25 (1H, m), 7.02 (2H, m), 4.17 (2H, q, 7.0Hz), 3.14 (3H, s), 3.12 (3H, s), 1.5 (3H, t, 7.0Hz); LC-MS rt 2.51 m/z 312 ES+.

*Step 2*: *[6-(4-Ethoxy-3-fluoro-phenyl)-quinazolin-4-yl]-(4-[1,2,4]triazol-1-yl-phenyl)-amine*

A mixture of formamidine from Step 1 (400 mg) and 4-triazolyl-aniline (214 mg, 1 eq) in AcOH (3ml) was heated to 80 ° for 2 h. Cooled, and the resulting solid was isolated by filtration, washed with sodium bicarbonate, water and MeCN and dried to give pure title compound.
$^1$H NMR δ 10.27 (1H, br s), 9.29 (1H, s), 8.86 (1H, s), 8.63 (1H, s), 8.26 (1H, s), 8.22 (1H, dd, J 8.75 2.5Hz), 8.10 (2H, d, J 7.50Hz), 7.88 (4H, m), 7.72 (1H, d, 7.5Hz), 7.34 (1H, t, 7.5Hz), 4.21 (2H, q, 7.5Hz), 1.41 (3H, t, 7.5Hz); LC-MS rt 2.53 m/z 427 ES+.

Example 9: {6-[3-Fluoro-4-(2-methoxy-ethoxy)-phenyl]-quinazolin-4-yl}-(4-[1,2,4]triazol-1-yl-phenyl)-amine

*Step 1: 4-Bromo-2 fluoro-1-(2-methoxy-ethoxy)-benzene*

A mixture of 4-fluorophenol (9.8 ml), powdered potassium carbonate (2 eq, 24 g) and bromoethyl methyl ether (1.1 eq, 20.16 ml) in acetone (60 ml) heated to reflux overnight. The cooled reaction mixture was diluted with water and extracted into EtOAc. The combined organic phases were washed with aqueous sodium carbonate, dried ($Na_2SO_4$) and concentrated to give the product as a mobile oil (22 g, quantitative).
$^1$H NMR ($CDCl_3$) δ 7.14 (2H, m), 6.84 (1H, t, J 8.85Hz), 4.09 (2H, m), 3.69 (2H, m), 3.38 (3H, s); LC-MS rt 2.67 m/z no ion.

*Step 2: 3-Fluoro-4-(2-methoxy-ethoxy)-phenylboronic acid*

To a solution of 4-bromo-2-fluoro-1-(2-methoxy-ethoxy)-benzene (4.98 g) in THF (40 ml) was added a small crystal of iodine and then Mg (730 mg, 1.5 eq) portion-wise. After addition, the mixture was heated to reflux for 4 h. The grey mixture was cooled to -78 ° trimethylborate (1.2 eq, 2.25 ml) added and allowed to warm overnight. 1N HCl (aq, 60 ml) was added and stirred for 30 min before being extracted into ether (2 x 50 ml). The combined organic phases were dried and concentrated and the resulting solid triturated with ether / petrol, isolated by filtration and dried to give the *title compound* as a cream solid (3.122 g, 73 %).
$^1$H NMR δ 8.04 (2H, br s), 7.53 (2H, m), 7.12 (1H, t, J 8.85Hz), 4.17 (2H, m), 3.67 (2H, m), 3.3 (3H, s); LC-MS rt 1.97 m/z 213 ES-.

*Step 3*: *N'-[3-Cyano-3'-fluoro-4'-(2-methoxy-ethoxy)-biphenyl-4-yl]-N,N-dimethyl-formamidine*

A mixture of 3-fluoro-4-(2-methoxyethoxy)-phenylboronic acid (1.83 g, 12 eq), intermediate 2 (2.32 g), potassium carbonate (1.29 g, 1.2 eq) in DMF / $H_2O$ (3:1, 40 ml) wass treated with dichloro(bis benzonitrile) palladium (II) (1 %, 30 mg) and stirred at ambient under $N_2$ for 4 h. The mixture was diluted with water (100 ml) and filtered then extracted with EtOAc (2 x 50ml) and these organic extracts added to the filter cake, dried and concentrated. The residue was dissolved in DCM, loaded onto a short column of silica and eluted portion-wise under suction with DCM/EtOH/$NH_3$ 400-200:8:1. On concentration, this gave a brown oil which was triturated with DCM/ ether/ petrol and on crystallization diluted with further petrol (60 ml). The title compound was isolated by filtration as a cream solid (1.665 g, 63 %).
$^1$H NMR ($CDCl_3$) 8 7.74 (1H, d, J 1.9Hz), 7.7(1H, s), 7.60 (1H, dd, J 8.85, 1.9Hz), 7.29 (3H, m), 7.1 (1H, d, J 8.2Hz), 7.05 (1H, d, J8.85Hz), 4.28 (2h, m), 3.84 (2H, m), 3.52 (3H, s), 3.16 (3H, s), 3.14 (3H, s); LC-MS rt 2.32 m/z 342 ES+.

*Step 4: {6-[3-Fluoro-4-(2-methoxy-ethoxy)-phenyl]-quinazolin-4-yl}-(4-[1,2,4]triazol-1-yl-phenyl)-amine*

A mixture of formamidine from Step 3 (594 mg) and 4-triazolyl-aniline (279 mg, 1 eq) in AcOH (6 ml) was heated to 125° for 2 h. On cooling and dilution with water (20 ml), a yellow precipitate was isolated by filtration, slurried with 1N NaOH and washed with water. After drying, this material was triturated with MeOH / water/ acetone ~ 10:5:5 to give,

after filtration and drying, a pale cream solid (510mg, 64%).
$^1$H NMR δ 10.1 (1H, br s), 9.27(1H, s), 8.8 (1H, s), 8.58 (1H, s), 8.24(1H, s), 8.18 (1H, d, J 8.85Hz), 8.03 (2H, d, J8.85Hz), 7.84 (4H, m), 7.68 (1H, d, J9.5Hz), 7.35 (1H, t, J 8.85Hz), 4.26 (2H, m), 3.72 (2H, m), 3.35 (3H, obscured by $H_2O$); LC-MS rt 2.4 m/z 457 ES+.

Example 10: [6-(3,4-Dimethoxy-phenyl)-quinazolin-4-yl]-(4-[1,2,4]triazol-1-yl-phenyl)-amine

*Step 1: N'-(3-Cyano-3',4'-dimethoxy-biphenyl-4-yl)-N,N-dimethyl-formamidine*

Intermediate 4 (2.52 g, 10 mmol) and 3,4-dimethoxyphenyl boronic acid (1.9 g, 1.2 eq) in iPrOH (30 ml) were treated with 2N aqueous sodium carbonate (10 ml) and tetrakis-(triphenylphosphine) palladium (0) (5 mg, 0.04 mol%) and heated with stirring at 60 °. After 2 hours the reaction was shown to be ca. 80 % complete by LCMS. The reaction mixture was then cooled and diluted with water (30 ml). The resulting solid was filtered off, washed with further water (2 x 20 ml) before drying by suction. The solid was re-slurried twice in ether (2 x 5 ml) sucked dry, and finally dried to give the biphenyl intermediate as an off white solid (2.24 g, 72 %).
$^1$H NMR δ 7.99 (1H, s) 7.91 (1H, d, J 1.9Hz), 7.79 (1H, dd, J 8.2, 1.9Hz), 7.2 (3H, m), 6.99 (1H, d, J 8.2Hz), 3.84 (3H, s), 3.78 (3H, s), 3.08 (3H, s) 3.01 (3H, s); LC-MS rt 2.31 m/z 309.

*Step 2: [6-(3,4-Dimethoxy-phenyl)-quinazolin-4-yl]-(4-[1,2,4]triazol-1-yl-phenyl)-amine*

Formamidine from step 1(2.96 g, 9.6 mmol) and 4-triazolylaniline (1.54 g, 9.6 mmol) in AcOH (10 ml) were heated to reflux for 3 h. The cooled solution was diluted with ether (200 ml) and the resulting precipitate filtered off. The filter cake was washed with ether, dried under suction then slurried with 2N NaOH (100 ml) with stirring for 30 minutes. The resulting solid was again isolated by filtration, washed with water and dried to give a pale yellow solid (3.81 g, 93 %).
$^1$H NMR δ10.11 (1H, s), 9.30 (1H, s), 8.80 (1H, s), 8.65 (1H, s), 8.26 (1H, s), 8.23 (1H, s), 8.10 (2H, d, J 10Hz), 7.92 (2H, d, J 10Hz), 7.88 (1H, d, J7.5Hz), 7.47 (2H, overlapping s), 7.16 (1H, d, J7.5Hz), 3.93 (3H, s), 3.85 (3H, s); LC-MS rt 2.17 m/z 425 ES+.

Example 11: [6-(1,3,4-Diethox-phenyl)-quinazolin-4-yl]-(4-[1,2,4]triazol-1-yl-phenyl)-amine

A mixture of Intermediate 8 (200 mg) and Intermediate 6 (490 mg) were combined with terakis(triphenylphosphine) palladium (0) (95 mg) in DME (3 ml) and sodium carbonate (1 ml) and heated to 100° overnight. The cooled mixture was diluted with water and extracted into DCM. The organic phases were combined, concentrated and partially purified by column chromatography with $CH_2Cl_2$/EtOH/ $NH_3$ (200:8:1) to give coupled material. A portion of this material (70 mg) was heated in acetic acid (1 ml) with 4-triazolylaniline (37 mg) at 80 °over 1 h. The mixture was concentrated, basified with sat. $NaHCO_3$ and the resulting precipitate isolated by filtration and washed with water and ether, dried then washed with EtOAc, MeCN, then ether and dried to give the title compound (32 mg, 34 %).
$^1$H NMR δ 10.4 (1H, br s), 9.27 (1H, s), 8.82 (1H, s), 8.6 (1H, s), 8.23 (1H, s), 8.16 (2H, d, J 8.85Hz), 7.95 (2H, d, J 8.85Hz), 7.44 (2H, m), 7.13 (1H, d, J 8.2Hz), 4.15 (4H, m), 1.37 (6H, m); LC-MS rt 2.44, m/z 453 ES+.

Example 12: {6-[4-(2-Dimethylamino-ethoxy)-phenyl]-quinazolin-4-yl}-(4-[1,2,4]triazol-1-yl-phenyl)-amine

*Step 1: N'-[3-Cyano-4'-(2-dimethylamino-ethoxy)-biphenyl-4-yl]-N,N-dimethyl-formamidine*

4-(2-dimethylamino-ethoxy)-boronic acid (prepared as per C. Zhou and R. C. Larock, Journal of Organic Chemistry, Vol. 70, No. 10, pp 3765, 915 mg, 1.2 eq), intermediate 2 (934 mg), and potassium carbonate (1.2 eq, 520 mg) in DMF/$H_2O$ (3:1, 20 ml) was treated with dichloro(bis benzonitrile) palladium (II) (1 %, 12 mg) and stirred at ambient under $N_2$ for 4 h. The mixture was concentrated and partitioned between water and EtOAc. The combined organic phases were dried and concentrated before being loaded onto an SPE (20 g, Si) and eluted portionwise under suction with a DCM - DCM/EtOH/$NH_3$ -200:8:1 gradient. On concentration, this gave a brown oil (1.0 g, quant).
$^1$H NMR ($CDCl_3$) δ 7.71 (1H, d, J2.5Hz), 7.64 (1H, s), 7.6 (1H, dd, J8.2, 2.5Hz), 7.44 (2H, d, 8.2Hz), 6.98 (3H, m), 4.1 (2H, m), 3.11 (3H, s), 3.08 (3h, s) 2.75 (2H, m), 2.29 (6H,s); LC-MS rt 1.71 m/z 337 ES+.

*Step 2: {6-[4-(2-Dimethylamino-ethoxy)-phexyl]-quinazolin-4-yl}-(4-[1,2,4]triazol-1-yl-phenyl)-amine*

A mixture of the formamidine from step 1 (528 mg) and 4-triazolylaniline (294 mg) in AcOH (5 ml) was heated to 125° for 3 h. After cooling, the mixture was diluted and basified with 1N NaOH (80 ml). The resulting cream ppt was isolated by filtration, and dried to give the title compound (495mg, 70%).

$^1$H NMR δ 10.3 (1H, br s), 9.34 (1H, s), 8.9 (1H, s), 8.67 (1H, s), 8.31 (1H, s), 8.23 (1H, dd, J 8.85, 1.9Hz), 8.15 (2H, d, J 8.85Hz), 7.92 (4H, m), 7.19 (2H, d, J 8.85Hz), 4.2 (2H, m), 2.72 (2H, m), 2.3 (6H, s); LC-MS rt 1.96 m/z 452 ES+.

Example 13: {6-[4-Pyridin-4-ylmethoxy)-phenyl]-quinazolin-4-yl}-(4-[1,2,4]triazol-1-yl-phenyl)-amine

*Step 1: 4-Amino-4'-(pyridin-4-ylmethoxy)-biphenyl-3-carbonitrile*

A mixture of Intermediate 5 (835 mg, 1.5 eq), Intermediate 7 (710 mg) and tetrakis(triphenylphosphine)palladium (0) (5 %, 263 mg) was heated in DME / 2N sodium carbonate (aq, 2:1, 15 ml) at 80 ° for 16 h. Aqueous workup between water and EtOAc gave a residue that was purified MPLC (35 g Si, 10-100 % EtOAc in petrol gradient elution over 30 min). This gave, on concentration, the title compound (310 mg, 45%).
$^1$H NMR ($CDCl_3$) δ 8.63 (2H, m), 7.5 (2H, m), 7:38 (3H, m), 6.99 (2H, d, J 8.65Hz), 6.79 (1H, d, J 8.65Hz), 5.127 (2H, s), 4.42 (2H, br s).

*Step 2: {6-[4-Pyridin-4-ylmethoxy)-phenyl]-quinazolin-4-yl}-(4-[1,2,4]-triazol-1-yl-phenyl)-amine*

Step 1 intermediate (310 mg) was treated with DMF-DMA (5 ml) in DMF (3 ml) at 80° for 3 h. The mixture was evaporated, dissolved in toluene and concentrated to a pale yellow solid which was dried overnight. A portion of this material (74 mg) was treated with 4-triazolylaniline (57 mg) in AcOH (1 ml) at 80° for 2 h. The cooled mixture was basified with aq sodium bicarbonate and the resulting precipitate filtered, washed with water, ether and MeCN to give the title compound (88 mg, 59 %).
$^1$H NMR δ 10.5 (1H, brs), 9.26 (1H, s), 8.87 (1H, s), 8.59 (3H, m), 8.23 (1H, s), 8.1 (3H, m), 7.84 (5H, m) 7.47 (5H, m), 7.18 (2H, m), 5.29 (2H, s); LC-MS rt 2.23 m/z 472 ES+.

Example 14: [6-(4-Morpholin-4-yl-phenyl)-quinazolin-4-yl]-(4-[1,2,4]triazol-1-yl-phenyl)-amine

*Step 1: N'-(3-Cyano-4'-morpholin-4-yl-biphenyl-4-yl)-N,N-dimethyl-formamidine*

A mixture of 4-morpholinylphenyl boronic acid (Maybridge, 992 mg, 1.5 eq), Intermediate 2 (955 mg) and tetrakis(triphenylphosphine)palladium (0) (5 %, 185 mg) was heated in DME / 2N sodium carbonate (aq, 2:1,12 ml) at 90° for 16 h. Aqueous workup between water and EtOAc gave a brown residue that was purified by SPE (Si, 20 g) with portionwise elution under suction with DCM/EtOH/$NH_3$ 600-200:8:1. This gave a brown solid that was triturated with DCM / petrol and filtered to give a light brown solid (430 mg, 40%).
$^1$H NMR($CDCl_3$) δ 7.45 (3H, m), 7.29 (2H, d, J 8.85 Hz), 6.81 (3H, m), 3.71 (4H, m), 3.03 (4H, m), 2.94 (3H, s), 2.91 (3H, s); LC-MS rt 2.16 m/z 335 ES+.

*Step 2: [6-(4-Morpholin-4-yl-phenyl)-quinazolin-4-yl]-(4-[1,2,4]triazol-1-yl-phenyl)-amine*

A mixture of the formamidine from step 1 (76 mg) and 4-triazolylaniline (24 mg) in AcOH (2 ml) was heated to 125 ° for 1 h. After cooling, the mixture was diluted and basified with 1N NaOH (20 ml). The resulting ppt was isolated by filtration, washed with DCM then triturated with MeOH /acetone. The filtrate was concentrated to give the title compound (22 mg) as a light green solid.
$^1$H NMR δ 10.45 (1H, br s), 9.3 (1H, s), 8.88 (1H, s), 8.6 (1H, s), 8.26 (1H, s), 8.14 (3H, m), 7.87 (5H, m), 7.12 (2H, d, J 8.85Hz), 3.78 (4H, m), 3.21 (4H, m) ; LC-MS rt 2.28 m/z 450 ES+.

Example 15:[6-(2,3-Dihyedro-[1,4]dioxin-6-yl)-quinazolin-4-yl]-(4-[1,2,4]triazol-1-yl-phenyl)-amine

*Step 1: N'-[2-Cyano-4-(2,3-dihydro-benzo[1,4]dioxin-6yl)-phenyl]-N,N-dimethyl-formamidine*

1,4-(Ethylenedioxy)benzene-6-boronic acid (Lancaster, 785 mg, 1.2 eq), Intermediate 2 (1.08 g) and tetrakis (triphenylphosphine)palladium (0) (205 mg) was heated in DME / 2N sodium carbonate (aq, 2:1,12 ml) at 80 ° for 18 h. Aqueous workup between water and EtOAc gave a brown solid that was purified by SPE (Si, 20 g) with portionwise elution under suction with DCM/EtOH/$NH_3$ 600-200:8:1. This gave a light brown solid (430 mg, 41 %).
$^1$H NMR($CDCl_3$) δ 7.68 (1H, d, J 2.5Hz), 7.63(1H, s), 7.56 (1H, dd, J 8.2, 2.5Hz), 6.96 (4H, m), 4.29 (4H, s), 3.1 (3H, s), 3.08 93H, s). LC-MS rt 2.22 m/z 308 BS+.

*Step 2: [6-(2,3-Dihydro-benzo[1,4]dioxin-6-yl)-quinazolin-4-yl]-(4-[1,2,4]triazol-1-yl-phenyl)-amine*

A mixture of the formamidine from step 1 (304 mg) and 4-triazolylaniline (75 mg) in AcOH (2 ml) was heated to 125° for 1.5 h. After cooling, the mixture was diluted and basified with 1N NaOH (20 ml). The resulting ppt was isolated by filtration and dried to give the title compound (136 mg, 71 %) as a yellow solid.
$^1$H NMR δ 10.07 (1H, s), 9.28 (1H, s), 8.78 (1H, s), 8.62 (1H,s), 8.25 (1H, s), 8.16 (1H, d, J 8.85Hz), 8.07 (2H, d, J 8.85Hz), 7.86 (2H, d, J 8.85Hz), 7.82 (1H, d, J 8.85Hz), 7.47 (1H, d, J 1.9Hz), 7.38 (1H, dd, J 8.2,1.9Hz), 7.03 (1H, d, J 8.85Hz) 4.32 (4H, s); LC-MS rt 2.35 m/z 423 ES+.

Example 16: [6-(2-Methoxy-pyrimidin-5-yl)-guinazolin-4-yl]-(4-[1,2,4]triazol-1-yl-phenyl)-amine

*Step 1: 2-Amino-5-(2-methoxy-pyrimidin-5-yl)-benzonitrile*

A mixture of Intermediate 3 (602 mg), 2-methoxypyfimidine-5-boronic acid (Frontier, 1.5 eq, 706 mg), and tetrakis(triphenylphosphine)palladium (0) (352 mg) was heated in DME / 2N sodium carbonate (aq, 2:1, 20 ml) at 100 ° for 12 h. Aqueous workup between water and EtOAc gave a brown solid that purified by SPE (Si, 20 g) by elution with DCM (4x10 ml) then EtOAc (4x10 ml), to give a solid that was triturated with DCM / petrol, and filtered to give a light brown solid (660 mg).
$^1$H NMR δ 8.85 (2H, s), 7.8 (1H, s), 7.6 (>3H, m), 6.88 (1H, d, J 8.85Hz), 6.3 (2H, s), 3.93 (3H, s); LC-MS rt 2.39 m/z 226 ES-.

*Step 2: [6-(2-Methoxy-pyrimidin-5-yl)-quinazolin-4yl]-(4-[1,2,4]triazol-1-yl-phenyl)-amine*

The product from step 1 (645 mg) was heated in DMF-DMA (4 ml) for 2 h. On cooling the mixture was concentrated and triturated with ether / petrol to give the formamidine as a solid (664 mg). A portion of this material (56 mg) was treated with 4-triazolyl-aniline (35 mg) in AcOH (1.5 ml) at 100 ° for 2 h, cooled and diluted with water (25 ml). Basified with NaOH, filtered and the solid dried to give the title compound (66 mg).
$^1$H NMR δ 10.04 (1H, s), 9.24 (1H, s), 9.13 (2H, s), 8.86 (1H, s), 8.63 (1H, s), 8.25 (2H, m), 8.04 (2H, m), 7.89 (3H, m), 3.99 (3H, s); LC-MS rt 2.32 m/z 395 ES-.

Example 17:(6-Thiophen-2-yl-quinazolin-4-yl)-(4-[1,2,4]triazol-1-yl-phenyl)-amine

*Step 1: N'-(2-Cyano-4-thiophen-2-yl-phenyl)-N,N-dimethyl-formamidine*

A mixture of Intermediate 2 (2 g), lithium chloride (1.42 g), dichlorobis-(triphenylphosphine) palladium (II) (0.235 g) and 2-(tributylstannyl)thiophene (2.74 g) in toluene (40 ml) was heated to 120 ° for 24 h. The cooled reaction mixture was concentrated and loaded onto a column of silica gel and eluted with DCM:MeOH (2.5 %) to give the title compound (0.4 g).
$^1$H NMR δ 8.02 (1H, s), 7.88 (1H, d, J 1.9Hz), 7.74 (1H, dd, J 8.85, 2.5Hz), 7.52(1H, s), 7.50 (1H, s), 7.2 (1H, d, J 8.85Hz), 7.11 (1H, t, J 4.4Hz), 3.09 (3H, s), 3.0 (3H, s); LC-MS rt 2.32 m/z 256 ES+.

*Step 2: (6-Thiophen-2-yl-quinazolin- 4-yl)-(4-[1,2,4] triazol- 1-yl- phenyl)-amine*

A mixture of formamidine from Step 1, (151 mg) and 4-triazolylaniline(100 mg) in AcOH (5 ml) were heated to 125 ° for 3 h. The cooled reaction mixture was basified with 2N NaOH and the precipitate isolated by filtration and purified by column chromatography on silica gel with DCM:MeOH (2.5%) as eluant. This gave the title compound (200 mg).
$^1$H NMR δ 10.1 (1H, s), 9.3 (1H, s), 8.83 (1H, s), 8.63 (1H, s), 8.26 (1H, s), 8.19 (1H, d, J 8.85Hz), 8.07 (2H, d, J 8.85Hz), 7.92 (2H, d, J 8.85Hz), 7.85 (1H, d, J 8.85Hz), 7.76 (1H, d, J 2.5Hz), 7.70 (1H, d, J 5Hz), 7.27 (1H, m); LC-MS rt 2.39 m/z 369 ES-.

Example 18: [6-(4-Methyl-thiophen-2-yl)-quinazolin-4-yl]-(4-[1,2,4]triazol-1-yl-phenyl)-amine

*Step 1: 2-Amino-5-(4-methyl-thiophen-2-yl)-benzonitrile*

A mixture of Intermediate 3 (1 g), 4-methyl-2-thienylboronic acid (Acros, 2 eq, 1.44 g), and tetrakis(triphenylphosphine)palladium (0) (586 mg) was heated in DME / 2N sodium carbonate (aq, 2:1, 30 ml) at 100 ° for 2.5 h. Aqueous workup between water and EtOAc gave a brown solid that was triturated with DCM/petrol, filtered and washed with ether to give a light brown solid (865 mg, 80 %).
$^1$H NMR δ 7.6 (1H ,d, J 2.52Hz), 7.54 (1h, dd, J 8.85, 2.5Hz), 7.16 (1H, s), 6.98 (1H, s), 6.80 (1H, d, J 8.85Hz), 6.25

(2H, br s), 2.19 (3H, s); LC-MS rt 2.91 m/z 215 ES+.

*Step* 2: *N'-[2-Cyano-4-(4-methyl-thiophen-2-yl)-phenyl]-N,N-dimethyl-formamidine*

The product from step 1 (850 mg) was heated in DMF-DMA (1.32 ml) for 1.5 h. On cooling the mixture was diluted with ether, filtered and washed with further ether to give the formamidine as a grey solid (564 mg). A portion of this material (54 mg) was treated with 4-triazolyl-aniline (35 mg) in AcOH (1.5 ml) at 100 ° for 2 h, cooled and diluted with water (25 ml). Basified with NaOH, filtered and the solid dried to give the title compound (54.4 mg, 70 %).
$^1$H NMR δ 10.0 (1H, s), 9.18 (1H, s) 8.67 (1H, s), 8.51 (1H, s), 8.14 (1H, s), 8.0 (3H, m), 7.8 (2H, d), 7.72 (1H, d, J 8.85Hz), 7.48 (1H, s), 7.15 (1H, s), 2.2 (3H, s); LC-MS rt 2.72 m/z 385 ES+.

Example 19: (6-Furan-2-yl-quinazolin-4-yl)-(4-[1,2,4]triazol-1-yl-phenyl)-amine

*Step1: N'-(2-Cyano-4-furan-2-yl-phenyl)-N,N-dimethyl-formamidine*

A mixture of Intermediate 2 (1 g), lithium chloride (0.71 g), dichlorobis(triphenylphosphine) palladium (II) (0.717 g) and 2-(tributylstannyl)furan (1.31 g) in toluene (25 ml) was heated to 90 ° for 24 h. The cooled reaction mixture was concentrated and loaded onto a column of silica gel and eluted with DCM, followed by DCM: MeOH (2.5 %) to give the product (0.32 g).
$^1$H NMR δ 8.05(1H, s), 7.91 (1H, d, J 1.9Hz), 7.8 (1H, dd, J 8.85,1.9Hz), 7.73 (1H, d, J 1.9Hz), 7.25 (1H, d, J 8.85Hz), 6.95 (1H, d, J 3.2Hz), 6.58 (1H, m), 3.1 (3H, s), 3.02 (3H, s); LC-MS rt 2.04 m/z 240 ES-.

*Step2: (6-Furan-2-yl-quinazolin-4-yl)-(4-[1,2,4]triazol-1-yl-phenyl)-amine*

The formamidine from Step 1 (100 mg) and 4-triazolylaniline (73 mg) in AcOH (3 ml) were heated at 125 ° for 3 h. The cooled reaction mixture was concentrated and purified by column chromatography on silica gel with DCM: MeOH (2.5 %) as eluant. This gave the product (54 mg).
$^1$H NMR δ 10.14 (1H, s), 9.28 (1H, s), 8.87 (1H, s), 8.62 (1H, s), 8.25 (2H, m), 8.06 (2H, m), 7.9 (4H, m), 7.16 (1H, m), 6.73 (1H, m); LC-MS rt 2.25 m/z 353 ES-.

Example 20: {6-[4-(2-Dimethylamino-ethoxy)-3-fluoro-phenyl]-quinazoline-4-yl}-(4-[1,2,4]triazole-1-yl-phenyl)amine

Step 1: [2-(4-Bromo-2-fluoro-phenoxy)-ethyl]-dimethyl-amine.

A solution of 4-Bromo-2-Fluorophenol (20mmol, 2.19ml) dimethylaminoethylchloride.HCl (25mmol, 3.6g), powdered potassium carbonate (80mmol), 11.06g) in dry ethanol (200ml) and dry toluene (200ml) heated to 80° overnight. The cooled reaction mixture was concentrated under vacuum and partitioned between ethyl acetate and water. The combined organic phases were washed with aqueous sodium carbonate, dried ($Na_2SO_4$) and concentrated to give the product as a yellow oil. Purification by chromatography with DCM : EtOH : NH3 (200:8:1) as eluant gave the desired compound as a pale oil (4.11 g, 78%).

*Step 2: 3-Fluoro-4-(2-dimethylamine-ethoxy)-phenylboronic acid*

To a solution of [2-(4-Bromo-2-fluoro-phenoxy)-ethyl]-dimetliyl-amine from step 1 (4.1g) in THF (40ml) was added a small crystal of iodine and then Mg (570mg, 1.5eq) portion-wise. After addition, the mixture was heated to reflux for 1h. The grey mixture was cooled to -78° trimethylborate (1.2eq), 2.1ml) added drop-wise and allowed to warm overnight. 1N HCl (aq, 60ml) was added and stirred for 30 min before being extracted into ether (2x50ml). The combined organic phases were dried and concentrated and the resulting solid triturated with acetone / ether, isolated by filtration and dried to give the *title compound* as a brown solid (2.6g, 73%). The product was used crude without further purification.

*Step 3: N'-[3-Cyano-3'-fluoro-4'-(2-methoxy-ethoxy)-biphenyl-4-yl]-N,N-dimethyl-formamidine*

A mixture of 3-fluoro-4-(2-dimethylamine-ethoxy)-phenylboronic acid (1.96g, 1.2eq), intermediate 2 (2.14g), potassium carbonate (1.19g, 1.2eq) in DMF / $H_2O$ (3:1, 20ml) was treated with dichloro(bis benzonitrile) palladium (II) (1%, 27mg) and stirred at ambient under $N_2$ for 2h. The mixture was diluted with water (200ml) and filtered then extracted with ethyl acetate (2x50ml) and these organic extracts added to the filter cake, dried and concentrated to a brown solid. The residue was dissolved in DCM, loaded onto a short column of silica and eluted portion-wise under suction with DCM / EtOH /$NH_3$ 400-200:8:1. On concentration, this gave a beige solid (1.88g, 74%).

LC-MS rt 1.75 m/z 355 ES+.

*Step 4: {6-[4-(2-Dimethylamino-ethoxy)-3-fluoro-phenyl]-quinazoline-4-yl}-(4-[1,2,4]triazol-1-yl-phenyl)amine*

To a solution of formamidine from Step 3 (250mg, 0.73mM) in acetic acid (2ml) was added the 4-triazolylaniline (117mg, 0.73mM). The reaction was heated to 125°C for 2hrs. The mixture was cooled down and 15ml of 2N NaOH was added. The product crashed out and was isolated by filtration. The solid was purified by column (SiOH) with DCM: EtOH:$NH_3$ 400-200:8:1 and isolated as a yellow solid (258mg, 75%).
$^1$H NMR ($D^6$-DMSO) δ 10.06 (1H, broad s), 9.29 (1H, s), 8.81 (1H, s) 8.63 (1H, s), 8.25 (2H, m), 8.09 (1H, s), 8.05 (1H, s), 7.83 (4H, m), 7.70 (1H, d, J 10Hz), 7.39 (1H, t, J 10Hz), 4.21 (2H, t, J 7.5Hz), 2.68 (2H, t, J 7.5Hz), 2.24 (6H , s).
LC-MS rt 1.99 m/z 470 ES+

Example 21: (6-Bromo-quinazolin-4-yl)-4-[1,2,4]triazol-1-yl-phenyl)-amine

A stirred solution of'N'-(4-bromo-2-cyano-phenyl)-N,N-dimethyl-formamidine (0.5g, 2mmol, 1eq) and 4-[1,2,4] triazol-1-yl-phenylamine (0.32g, 2mmol, 1eq in acetic acid (4mL) was heated to reflux for 2 hours then allowed to cool. After cooling, addition of diethylether afforded a yellow precipitate. The precipitate was collected by filtration, washed with diethylether then dried in vacuo to afford the desired compound as the acetate salt. (0.53g, 62%). $^1$H-NMR (DMSO-$d_6$) δ. 1.88 (s, 3H), 7.77 (d, 1H), 7.90 (d, 2H), 8.02 (dd, 1H), 8.08 (d, 2H), 8.25 (s, 1H), 8.68 (s, 1H), 8.90 (d, 1H), 9.29 (dd, 1H), 10.04 (s, 1H). LC-MS rt 2.30 m/z 368 ES+.

Example 22: *N*-(2-{2-fluoro-4-[4-(4-[1,2,4]-triazol-1-yl-phenylamino)quinazoline-6-yl}ethyl)-*N'*,*N'*,*N'*-trimethylethane-1,2-diamine

A solution of Intermediate 11 (96mg, 0.23mmol, leq), and 4-(1*H*-1,2,4-triazol-1-yl)aniline (38mg, 0.24mmol, 1.05mmol) in acetic acid (2mL) was heated at reflux. After 1h the reaction was allowed to cool to room temperature, concentrated *in vacuo* and treated with a saturated aqueous solution of $K_2CO_3$ until effervescence ceased. The resulting mixture was then concentrated *in vacuo* to dryness to yield a brown residue. The residue was purified using flash column chromatography, eluting initially with 200:8:1 and then 100:8:1 $CH_2Cl_2$:EtOH:$NH_3$. The title compound was isolated as an off-white solid (48mg, 40%). $R_f$ = 0.39 (40:8:1 $CH_2Cl_2$:EtOH:$NH_3$). $^1$H-NMR (DMSO-$d_6$) 2.11 (s, 6H), 2.17 (s, 3H), 2.43 (m, 2H), 2.68 (t, 2H), 4.08 (t, 2H), 7.23 (m, 1H), 7.56 (m, 1H), 7.75 (m, 3H), 7.93 (m, 2H), 8.08 (m, 2H), 8.50 (s, 1H), 8.68 (s, 1H), 9.14 (s, 1H), 9.92 (br. s, 1H). LC-MS rt 2.08 m/z 527 ES+

Example 23: [6-(3-Isopropoxy-4-methoxy-phenyl)-quinazolin-4-yl]-(4-[1,2,4]triazol-1-yl-phenyl)-amine

To a solution of Intermediate 18 (60mg, 0.18mmol) in acetic acid (1ml) was added 4-triazolylaniline (35mg, 0.22mmol) and the solution was stirred at 80°C over 2 hours. LCMS analysis showed consumption of starting material. Reaction mixture was cooled and the excess acetic acid removed by evaporation under reduced pressure. The residues were treated with sat. aq. Sodium hydrogen carbonate until cessation of effervescence. The resulting precipitate was filtered and the solids obtained dried under vacuum.
Purification by preparative chromatography furnished the desired compound as an off-white solid (18mg, 22%)
$^1$H NMR ($D^6$-DMSO) 10.21 (1H, bs), 9.07 (1H, s), 8.57 (1H, s), 8.42 (1H, s), 8.04 (1H, s), 8.00 (1H, dd, J 8.75Hz, 1.5Hz), 7.90 (2H, d, J 9Hz), 7.72 (1H, s), 7.68 (2H, d, J 3.25Hz), 7.27 (2H, s), 6.96 (1H, d, J 9Hz), 4.53 (1H, quin, J 6Hz), 3.64 (3H, s), 1.12 (6H, d, J 6.25Hz)
LC-MS rt 2.48 m/z 454 ES+

Example 24: 6-(4-Fluoro-3-isopropoxy-phenyl)-quinazolin-4-yl]-(4-[1,2,4]triazol-1-yl-phenyl)-amine

To a solution of Intermediate 19 (95mg, 0.29mmol) in acetic acid (1ml) was added 4-triazolylaniline (56mg, 0.35mmol) and the solution was stirred at 80°C over 2 hours. LCMS analysis showed consumption of starting material. Reaction mixture was cooled and the excess acetic acid removed by evaporation under reduced pressure. The residues were treated with sat. aq. Sodium hydrogen carbonate until cessation of effervescence. The resulting precipitate was filtered and the solids obtained dried under vacuum.
Purification by preparative chromatography furnished the desired compound as an off-white solid (33mg, 25%)
$^1$H NMR ($D^6$-DMSO) 10.24 (1H, bs), 9.27 (1H, s), 8.82 (1H, s), 8.64 (1H, s), 8.36 (1H, s), 8.24 (1H, s), 8.22 (1H, dd, J 9Hz, 1.75Hz), 8.09 (2H, d, J 9Hz), 7.92 (3H, m), 7.64 (1H, dd, J 8.25Hz, 2Hz), 7.46 (1H, m), 4.87 (1H, quin, J 6Hz), 1.36 (6H, d, J 6Hz) LC-MS rt 2.66 m/z 441 ES+

Example 25: {6-[4-(2-Dimethylamino-ethoxy)-3-fluorophenyl]-quinazoline-4-yl}-(4-[1,2,4]triazole-1-yl-pheny)amine

Step 1:4-Bromo-1-(3-chloro-propoxy)-2-fluoro-benzene

A mixture of 2-fluoro-4-bromophenol (162.6mmol, 31.07g) powdered potassium carbonate (731.7mmol, 103g), 1-bromo-3-chloropropane (270mmol), 26.6ml) in acetonitrile (250ml) was heated to reflux for 3h. The cooled reaction mixture was filtered through celite and concentrated under vacuum. This gave a pale oil (43.5g, 100%).
$^1$H NMR ($CDCl_3$) δ 6.99 (2H, t, J 8.75Hz), 6.67 (1H, t, J 8.75Hz), 3.97 (2H, t, J 6.00Hz ), 3.57 (2H, t, J 6.00Hz), 2.04 (2H, m).

*Step 2: 1-[3-( 4-bromo- 2 -fluoro-phenoxy)-propyl]-pyrrolidine*

A mixture of 4-Bromo-1-(3-chloro-propoxy)-2-fluoro-benzene from step1 (0.046mM,11.94g) and pyrrolidine (0.138mM,11.43ml) in DMA (50ml) was heated to reflux for 48h. The cooled reaction mixture was partitioned between ethylacetate and saturated $NaHCO_3$. The combined organic phases were dried over magnesium sulphate, concentrated and a brown oil was isolated (13.5g, 98%).
$^1$H NMR ($CDCl_3$) δ 7.11 (2H, m), 6.79 (1H, t, J 8.75Hz), 4.01 (2H, t, J 6.25Hz), 2.52 (2H, t, J 7Hz), 2.42 (6H, m), 1.95 (3H, m), 1.69 (1H, m, obscured by H2O).

*Step 3: 3-Fluoro-4-(3-pyrrolidine-1-yl-propoxy)phenylborortic acid*

A solution of 1-[3-(4-bromo-2-fluoro-phenoxy)-propyl]pyrrolidine from step2 (5.31g, 17.57mmol) in THF (10ml) was added drop-wise to a stirred slurry of Mg (640mg, 1.5eq) in THF (2ml) and a small crystal of iodine. After addition, the mixture was heated to reflux for 3h. The grey mixture was cooled to -78° trimethylborate (1.2eq, 2.4ml) added drop-wise and allowed to warm overnight. 2N HCl (aq, 60ml) was added and stirred for 30 min before being extracted into ether (2x50ml). The combined organic phases were dried, concentrated and the resulting brown oil triturated with acetone / ether / petrol, to give a brown solid. The product was used crude without further purification.
$^1$H NMR ($d_6$-DMSO) δ 8.08 (2H, broad s), 7.55 (1H, t, J 8.75Hz), 7.15 (2H, m), 4.04 (2H, t, J 6.25Hz), 2.50 (3H, m), 2.45 (6H, m), 1.84 (3H, m).

*Step 4: N'-[3-Cyano-3'-fluoro-4'-(3-pyrrolidin-1-yl-propoxy)-biphenyl-4-yl]-N,N-dimethyl-formamidine*

A mixture of 3-fluoro-4-(3-pyrrolidine-1-yl-propoxy)-phenylboronic acid (3.074g, 2eq), intermediate 2 (1.72g, 1eq), potassium carbonate (0.954g, 1.2eq) in DMF / $H_2O$ (3:1, 40ml) was treated with dichloro(bis benzonitrile) palladium (II) (2%, 44mg) and stirred at ambient under $N_2$ for 18h. The mixture was diluted with water (200ml) and extracted with ethyl acetate (2x50ml). After drying over magnesium sulphate these organics phases were concentrated to a brown gum. The residue was dissolved in DCM, loaded onto a short column of silica and eluted portion-wise under suction with DCM/ EtOH/$NH_3$ 200:8:1 to 50:8:1 to give after concentration a light brown solid (1.63g, 71%).
LC-MS rt 1.99 m/z 395 ES+

Step 5: *{6-[3-fluoro-4-(3-pyrrolidin-1-yl-propoxy)-phenyl]-quinazoline-4-yl}-(4-[1,2,4]triazole-1-yl-phenyl)amine*

To a solution of formamidine from Step 4 (257mg, 0.653mM) in acetic acid (3ml) was added the 4-triazolylaniline (115mg, 0.73mM). The reaction was heated to 125°C for 1h. The mixture was cool down basified with 15ml of 2N NaOH and extracted with ethylacetate. After drying over magnesium sulphate these organics phases were concentrated to a yellow solid. The solid was triturated with DCM / Et2O / petrol and isolated as a yellow solid (163mg, 44%).
$^1$H NMR ($D_6$-DMSO) δ 10.07 (1H, broad s), 9.29 (1H, s), 8.83 (1H, s), 8.64 (1H, s) 8.26 (2H, m), 8.07 (2H, m), 7.85 (5H, m), 7.69 (1H, d, J 10Hz), 7.35 (1H, dd, J 10Hz, 5Hz), 4.22 (2H, t, J 7.5Hz), 2.46 (6H, m, obscured by H2O), 2.08 (2H, t, J 7.5Hz), 1.70 (4H , m).
LC-MS rt 2.13 m/z 509 ES+

Example 26: {6-[-3-fluoro-4-(3-morpholin-4-yl-propoxy)- phenyl]-quinazoline-4-yl}-(4-[1,2,4]triazole-1-yl-phenyl)amine

*Step 1: 4-Bromo-1 -(3-chloro-propoxy)-2-fluoro-benzene*

A mixture of 2-fluoro-4-bromophenol (162.6mmol, 31.07g), powdered potassium carbonate (731.7mmol, 103g), 1-bromo-3-chloropropane (270mmol, 26.6ml) in acetonitrile (250ml) was heated to reflux for 3h. The cooled reaction mixture was filtered through celite and concentrated under vacuum. This gave a pale oil (43.5g, 100%).
$^1$H NMR ($CDCl_3$) δ 6.99 (2H, t, J 8.75Hz), 6.67 (1H, t, J 8.75Hz), 3.97 (2H, t, J 6.00Hz ), 3.57 (2H, t, J 6.00Hz), 2.04 (2H, m).

*Step 2*: *4-[3-(4-bromo-2-fluoro-phenoxy)-propyl]-morpholine*

A mixture of 4-Bromo-1-(3-chloro-propoxy)-2-fluoro-benzene from step 1 (0.038mM,10.02g) and morpholine (0.114mM,10.1ml) in DMA (50ml) was heated to reflux for 48h. The cooled reaction mixture was partitioned between ethyl acetate and saturated $NaHCO_3$. The combined organic phases were dried over magnesium sulphate, concentrated and a brown oil was isolated (12g, 98%).
$^1H$ NMR ($CDCl_3$) δ 7.36 (1H, m), 7.32 (1H, m), 6.98 (1H, t, J 8.75Hz), 4.24 (2H, t, J 6.25Hz), 3.87 (4H, m), 2.62 (6H, m), 2.12 (2H, m).
LC-MS rt 1.98 m/z 320 ES+

*Step 3: 3-Fluoro-4-(3-morpholine-1-yl-propoxy)-phenylboronic acid*

A solution of 4-[3-(4-bromo-2-fluoro-phenoxy)-propyl]-morpholine from step 2 (5.08g, 15.96mmol) in THF (10ml) was added drop-wise to a stirred slurry of Mg (582mg, 1.5eq) in THF (2ml) and a small crystal of iodine. After addition, the mixture was heated to 90° for 1h. The grey mixture was cooled to -78° trimethylborate (1.2eq, 2.15ml) added drop-wise and allowed to warm overnight. 2N HCl (aq, 60ml) was added and stirred for 30 min before being extracted into ether (2x50ml). Then triethylamine was added to the aqueous layer and extracted with ethyl acetate and ether. The combined organic phases were dried, concentrated to give a brown oil (3.8g, 84%). The product was used crude without further purification.
$^1H$ NMR ($D_6$-DMSO) δ 8.08 (2H, broad s), 7.55 (1H, t, J 8.75Hz), 7.15 (2H, m), 4.04 (2H, t, J 6.25Hz), 3.57 (4H, m), 2.37 (6H, m), 1.84 (2H, m).
LC-MS: rt 1.95 m/z 284 ES+.

*Step 4: N'-[3-Cyano-3'-fluoro-4'-(3morpholin-1-yl-propoxy)-biphenyl-4-yl]-N,N-dimethyl-formamidine*

A mixture of 3-fluoro-4-(3-morpholine-1-yl-propoxy)-phenylboronic acid (3.8g, 1.5eq), intermediate 2 (2.67g, 1eq), potassium carbonate (1.48g, 1.2eq) in DMF / $H_2O$ (3:1, 40ml) was treated with dichloro(bis benzonitrile) palladium (II) (2%, 68mg) and stirred at ambient under $N_2$ for 18h. The mixture was diluted with water (200ml) and extracted with EtOAc (2x50ml). After drying over magnesium sulphate these organics phases were concentrated to a brown gum. The residue was dissolved in DCM, loaded onto a short column of silica and eluted portion-wise under suction with DCM / EtOH / $NH_3$ 200:8:1 to give after concentration a mobile brown solid (3.16g, 85%). The product solidified on standing.
$^1H$ NMR ($CDCl_3$) δ 7.79 (1H , broad s), 7.42 (1H , m), 7.02 (1H, m), 6.79 (3H, m) 3.88 (2H, m), 3.49 (4H, m), 2.87 (2H, d, J 5.5Hz), 2.73 (3H, s), 2.66 (3H, s), 2.30 (5H, m), 1.75 (2H, m).
LC-MS rt 1.91 m/z 411 ES+

*Step 5*: *{6-[3-fluoro-4-(3-morpholin-4-yl-propoxy)-phenyl]-quinazoline-4-yl}-(4-[1,2,4]triazole-1-yl-phenyl)amine*

To a solution of formamidine from Step 4 (429mg, 1.045mmol) in acetic acid (3ml) was added the 4-triazolylaniline (184mg, 1.15mmol). The reaction was heated to 125°C for 1h. The mixture was cooled down basified with 15ml of 2N NaOH and the resulting precipitate isolated by filtration then dissolved in DCM and purified by column chromatography on silica with DCM / EtOH / $NH_3$ 400:8:1 up to 50:8:1 to give after concentration a cream solid (136mg, 25%).
LC-MS rt 2.1 m/z 526 ES+.
$^1H$ NMR ($D_6$-DMSO) δ 10.06 (1H, broad s), 9.29 (1H, s), 8.82 (1H, s), 8.65 (1H, s) 8.21 (2H, m), 8.07 (2H, m), 7.83(4H, m), 7.69 (1H d, J 10Hz), 7.32 (1H, dd, J 10Hz, 5Hz), 4.17 (2H, t, J 7.5Hz), 3.58 (4H, t, J 2.5Hz), 3.34 (2H, m, obscured by H2O), 2.40 (4H, t, J 2.5Hz), 1.92 (2H, t, J 7.5Hz).

<u>Example 27: [6-(3,4-Dimethoxy-phenyl)-quinazolin-4-yl]-(2-methyl-4-[1,2,4]triazol-1-yl-phenyl)-amine</u>

To a carousel tube was added the formamidine (example 10 step 1) (0.05g, 0.16mmol), Intermediate 21 (0.031g, 0.18mmol) and AcOH (1.5ml). The mixture was left to stir at 120°C for 3 hr. The mixture was allowed to cool and concentrated to dryness and put on a silica column and eluted with 2.5% MeOH: DCM. A white solid was isolated 0.015g, (21%).
$^1H$ n.m.r ($D_6$-DMSO) 9.69 (1H, s), 9.10 (1H, s), 8.54 (1H, s), 8.23 (1H, s), 8.06 (1H, s), 8.02 (1H, m), 7.69-7.24 (6H, m), 6.94 (1H, d, J = 8.85Hz), 3.71 (3H, s), 3.64 (3H, s), 2.11 (3H, s);
LC-MS rt 2.18 m/z 438 ES+

Example 28: 1-(4-{6-[3-Fluoro-4-(2-methoxy-ethoxy)-phenyl]-quinazolin-4-ylamino}-phenyl)-5-methyl-1H-[1,2,4]triazole-3-carboxylic acid

To a solution of Intermediate 22 (55mg, 0.16mM) in acetic acid (3ml) was added the intermediate 24 (42mg, 0.19mM). The reaction was heated to 100°C for 2hrs. The mixture was cooled down and 15ml of 2N NaOH was added. The product crashed out and was isolated by filtration. The solid was purified by preparative HPLC and isolated as a white solid (26mg, 32%).
$^1$H NMR ($D_6$-DMSO) δ 10.15 (1H, broad s), 8.82 (1H, s), 8.56 (1H, s) 8.15 (1H, d, J 7.5Hz), 8.00 (2H, d, J 7.5Hz), 7.80 (2H, m), 7.68 (1H, d, J 7.5Hz), 7.56 (2H, d, J 7.5Hz), 7.32 (1H, t, J 7.5Hz), 4.27 (3H, s), 3.73 (3H, s); LC-MS: rt 2.31 m/z 512 $ES^-$.

Example 29: 1-(4-{6-[3,4-Dimethoxy-phenyl]-quinazolin-4-ylamino}-phenyl)-5-methyl-1H-[1,2,4]triazole-3-carboxylic acid

To a solution of Intermediate 23 (50mg, 0.16mM) in acetic acid (3ml) was added the Intermediate 24 (42mg, 0.19mM). The reaction was heated to 100°C for 2hrs. The mixture was cooled down and 15m of 2N NaOH was added. The product crashed out and was isolated by filtration. The solid was purified by preparative HPLC and isolated as a white solid (27mg, 34%).
$^1$H NMR ($D_6$-DMSO) δ 10.11 (1H, broad s), 8.82 (1H, s), 8.68 (1H s) 8.26 (1H, dd, J 10Hz, J 2.5Hz), 8.14 (2H, d, J 10Hz), 7.87 (1H, d, J 10Hz), 7.67 (2H, d, J 10Hz), 7.46 (2H, dd, J 10Hz, J 2.5Hz), 7.14 (1H,d, J 10Hz), 3.93 (3H, s), 3.85 (3H, s); LC-MS: rt 2.16 m/z 482 $ES^+$.

Example 30: 1-{4-[6-(3,4-Dimethoxy-phenyl)-quinazolin-4-ylamino]-phenyl}-5-methyl-1H-[1,2,4]triazole-3-carboxylic acid dimethylamide

To a solution of Intermediate 23 (50mg, 0.16mm) in acetic acid (3ml) was added Intermediate 25 (49mg, 0.19mM). The reaction was heated 2hrs to 100°C. The mixture was cooled down and 15ml of 2N NaOH was added. The product crashed out and the solid was collected by filtration. The solid was purified by preparative HPLC and isolated as a white solid (23mg, 28%).
$^1$H NMR ($D_6$-DMSO) δ 10.09 (1H, broad s), 8.82 (1H, s), 8.67 (1H, s) 8.26 (1H, dd, J 8.75Hz, J 1.5Hz), 8.13 (2H, d, J 8.75Hz), 7.87 (1H, d, J 8.7Hz), 7.66 (2H, d, J 8.7Hz), 7.46 (2H, m), 7.14 (1H,d, J 8.7Hz), 3.93 (3H, s), 3.85 (3H, s), 3.15 (3H, s), 3.03 (3H, s), 2.55 (3H, s); LC-MS: rt 2.22 m/z 510 $ES^+$

Example 31: 1-(4-{6-[3-Fluoro-4-(2-methoxy-ethoxy)-phenyl]-quinazolin-4-ylamino}-phenyl)-5-methyl-1H-[1,2,4]triazole-3-carboxylic acid methylamide

*Step1: 5-Methyl-1-(4-nitrophenyl)-1H-[1,2,4]triazole-3-carboxylic acid methylamide.*

To a solution of 5-Methyl-1-(4-nitrophenyl)-1H-1,2,4-triazole-3-carboxylic acid (Key Organics, 400mg, 1.61mM) and Hunig's base (667ul, 3.86mM) in DCM:DMF (6ml: 1ml) at -10°C was added drop-wise isobutylchloroformate (250ul, 1.93mM). The reaction was stirred 30min. at -10°C then slowly a 2M solution of methylamine (965ul, 1.93mM) was added. The mixture was allowed to warm-up to r.t. and stirred for another hour. The crude mixture was washed with sat. $NaHCO_3$ extracted and dried over $MgSO_4$. After evaporation the solid obtained was used directly in the next reaction without further purification (374mg, 89%). LC-MS: rt 2.01 m/z 261 $ES^+$.
$^1$H NMR (d-DMSO) δ 8.42 (2H, d, J 7Hz), 7.95 (2H, d, J 7Hz), 2.73 (3H, d, J 4.75Hz), 2.60 (3H, s).

*Step 2: 1-(4-Amino-phenyl)-5-methyl-1H-[1,2,4]triazole-3-carboxylic acid methylamide.*

A solution of 5-Methyl-1-(4-nitrophenyl)-1H-[1,2,4]triazole-3-carboxylic acid methylamide from step 1 (100mg, 0.38mmol) in methanol (8ml) was injected at a 1ml/min rate into an hydrogenator "the H-Cube" that combines endogenous hydrogen generation with a disposable cartridge system (Pd/C), temperature was set up to 25°C at atmospheric pressure. The solution obtained was concentrated to give a white solid (86mg, 97%). LC-MS: rt 1.15 m/z 231 $ES^+$.
$^1$H NMR (d-DMSO) δ 8.61 (1H, broad s), 8.54 (2H, d, J 7Hz), 7.12 (2H, d, J 7Hz), 2.73 (3H, d, J 4.75Hz), 2.60 (3H, s).

*Step3: 1-(4-{6-[3-Fluoro-4-(2-methoxy-ethoxy)-phenyl]-quinazolin-4-ylamino}-phenyl)-5-methyl-1H-[1,2,4]triazole-3-carboxylic acid methylamide.*

To a solution of Intermediate 22 (50mg, 0.16mM) in acetic acid (3ml) was added 1-(4-Amino-phenyl)-5-methyl-1H-[1,2,4]triazole-3-carboxylic acid methylamide from step 2 (44mg, 0.19mM). The reaction was stirred 2hrs at a 100°C. The mixture was cooled down and 15ml of 2N NaOH was added. The product crashed out and the solid was collected by filtration. The solid was purified by preparative HPLC and isolated as a white solid (6mg, 8%). LC-MS: rt 2.33 m/z 528 $ES^+$.
$^1$H NMR (d-DMSO) δ 10.02 (1H, broad s), 8.74 (1H, s), 8.56 (1H, s) 8.21 (1H, dd, J 8.75Hz, J 1.5Hz), 8.01(2H, d, J 8.7Hz), 7.79 (2H, m), 7.60 (2H, d, J 8.7Hz), 7.57 (1H, s), 7.27 (1H, t, J 8.7Hz), 4.15 (2H, t, J 4.5Hz), 3.62 (2H, t, J 4.5Hz), 3.24 (3H, s), 2.67 (3H, d, J 4.7Hz), 2.44 (3H, s).

Example 32: 1-(4-{6-[3-Fluoro-4-(2-methoxy-ethoxy)-phenyl]-quinazolin-4-ylamino}-phenyl)-5-methyl-1H-[1,2,4]triazole-3-carboxylic acid dimethylamide

To a solution of Intermediate 22 (50mg, 0.16mM) in acetic acid (3ml) was added the intermediate 25 (47mg, 0.19mM). The reaction was heated 2hrs to 100°C. The mixture was cooled down and 15ml of 2N NaOH was added. The product crashed out and the solid was collected by filtration. The solid was purified by preparative HPLC and isolated as a white solid (23mg, 27%).
$^1$H NMR (d-DMSO) δ 10.09 (1H, broad s), 8.83 (1H, s), 8.66 (1H, s) 8.22 (1H, dd, J 7.5Hz, J 1.5Hz), 8.14(1H, d, J 5Hz), 8.10(1H, s), 7.85 (2H, m), 7.70 (2H, s), 7.66 (1H, s), 7.33 (1H,dd, J 10Hz, J 7.5Hz), 4.25 (2H, t, J 5Hz), 3.71 (2H, t, J 5Hz), 3.14 (3H, s), 3.01 (3H, s), 2.67 (3H, s), 2.44 (3H, s); LC-MS: rt 2.37 m/z 542 $ES^+$.

Example 33: (6-Bromo-quinazolin-4-yl)-(2-methyl-4-[1,2,4]triazol-1-yl-phenyl-amine

To a carousel tube was added 6-bromo-4-chloro-quinazoline (0.1g, 0.41mmol), Intermediate Y (0.078g, 0.45mmol) and $CH_3CN$ (anhydrous, 4ml) and the mixture was stirred at 90°C under nitrogen for 24 hr. A orange precipitate had formed which was filtered off and washed with water, 1N NaOH and water again and dried under a vacuum at 40°C. Isolated 0.1g (51 %) of solid.
$^1$H n.m.r ($D_6$-DMSO) 9.26 (1H, s), 9.05 (1H, s), 8.76 (1H, s), 8.20 (1H, s), 8.17 (1H, d, J =1.26Hz), 7.85 (2H, bs), 7.81 (1H, s), 7.74 (1H, dd, J = 8.85Hz, 2.53Hz), 7.47 (1H, d, J = 8.21Hz), 2.25 (3H, s); LC-MS rt 2.19 m/z 382 ES+

Example 34: [1-(4-{6-[3-Fluoro-4-(2-methoxy-ethoxy)-phenyl]quinazolin-4-ylamino}-phenyl)-5-methyl-1H-[1,2,4]triazole-3-yl]-1-(4-methyl-piperazin-1-yl)methanone

*Step 1: [5-Methyl-1-(4-nitro-phenyl-)-1H-1,2,4]triazol-3yl]-(4-methyl-piperazin-1-yl)-methanone.*

To a solution of 5-Methyl-1-(4-nitrophenyl)-1H-1,2,4-triazole-3-carboxylic acid (Key Organics, 400mg, 1.61mM) and Hunig's base (667ul, 3.86mM) in DCM:DMF (6ml:1ml) at -10°C was added drop-wise isobutylchloroformate (250ul, 1.93mM). The reaction was stirred 30min. at -10°C then slowly methylpiperazine (214ul, 1.93mM) was added. The mixture was allowed to warm-up to r.t. and stirred for another hour. The crude mixture was washed with sat. $NaHCO_3$ extracted and dried over $MgSO_4$. After evaporation the solid obtained was used directly in the next reaction without further purification (335mg, 63%). LC-MS: rt 1.26 m/z 330 $ES^+$.
$^1$H NMR ($D_6$-DMSO) δ 8.39 (2H, d, J 7Hz), 7.92 ((2H, d, J 7Hz), 3.58 (4H, m), 3.30 (4H, m), 2.58 (3H, s), 2.15 (3H, s).

*Step 2 : [1-(4-Amino-phenyl)-5-methyl-1H-[1,2,4]triazol-3-yl]-(4-methyl-piperazin-1-yl) methanone.*

A solution of [5-Methyl-1-(4-nitro-phenyl-)-1H-[1,2,4]triazol-3-yl]-(4-methyl-piperazin-1-yl)-methanone from step 1 (100mg, 0.302mmol) in methanol (8ml) was injected at a 1ml/min rate into an hydrogenator "the H-Cube" that combines endogenous hydrogen generation with a disposable cartridge system (Pd/C), temperature was set up to 25°C at atmospheric pressure.
The solution obtained was concentrated to give a white solid (87mg, 96%). LC-MS: rt 2.22 m/z 301$ES^+$.
$^1$H NMR ($D_6$-DMSO) δ 8.60 (1H, broad s), 7.51 (2H, d, J 7Hz), 7.09 ((2H, d, J 7Hz), 3.58 (4H, m), 3.30 (4H, m), 2.58 (3H, s), 2.15 (3H, s).

*Step3: [1-(4-{6-[3-Fluoro-4-(2-methoxy-ethoxy)-phenyl]-quinazolin-4-ylamino}-phenyl)-5-tnethyl-1H-[1,2,4]triazole-3-yl]-(4-methyl-piperazin-1-yl)methanone.*

To a solution of Intermediate 22 (55mg, 0.16mM) in acetic acid (3ml) was added [1-(4-Amino-phenyl)-5-methyl-1H-[1,2,4]-triazol-3-yl]-(4-methyl-piperazin-1-yl) methanone from step 2 (58mg, 0.19mM). The reaction was heated 2hrs to 100°C. The mixture was cooled down and 15ml of 2N NaOH was added. The product crashed out and the solid was collected by filtration. The solid was purified by preparative HPLC and isolated as a white solid (25mg, 26%). LC-MS: rt

2.11 m/z 597 ES+.
$^1H$ NMR ($D_6$-DMSO) δ 10.32 (1H, broad s), 9.05 (1H, s), 8.88 (1H, s) 8.45 (1H, dd, J 7.5Hz, J 1.5Hz), 8.35(1H, d, J 5Hz), 8.31(1H, s), 8.08 (2H, m), 7.92 (2H, s), 7.86 (1H, s), 7.58 (1H,dd, J 10Hz, J 7.5Hz), 4.47 (2H, t, J 5Hz), 3.95 (2H, t, J 5Hz), 3.88 (4H, m), 3.57 (3H, obscured by $H_2O$), 3.53 (4H, obscured by $H_2O$), 2.78 (3H , s), 2.43 (3H, s).

Example 35: 2-Methoxy-4-[4-(4-[1,2,4]triazol-1-yl-phenylamino)-quinazolin-6-yl]-phenol

*Step 1: N'-(3-Cyano-4'-hydroxy-3'-methoxy-biphenyl-4-yl)-N,N-dimethyl-formamidine*

A mixture of boronate (Aldrich, 2-methoxy-4-(4,4,5,5-tetramethyl-1,3,2-dioxaborolan-2-yl)phenol, 500mg, 1.2eq) and Intermediate 2 (1eq, 498mg), potassium carbonate (1.2eq, 276mg) in DMF/H2O (3:1, 12ml) was treated with Pd $Cl_2(PhCN)_2$ (2%, 13mg) and stirred at rt under N2 for 12h. The mixture was diluted with water (50ml) and filtered through a plastic frit under suction. The filter cake was washed with diethyl; ether and dried in vacuo to give a wine coloured solid (390mg, 79%),
$^1H$ NMR ($D_6$-DMSO) δ 3.18 (6H, d); 3.98 (3H, s); 6.94 (1H,d), 7.2 (1H, d); 7.32 (3H, m); 7.88 (1H, d); 7.99 (1H, s); 8.11 (1H, s); 9.23 (1H, s); LCMS rt 1.93 m/z 295 ES+

*Step* 2: *2-Methoxy-4-[4-(4-[1,2,4] triazol-1-yl-phenylamino)-quillazolin-6-yl]-phenol*

A mixture of N-(3-Cyano-4'-hydroxy-3'-methoxy-biphenyl-4-yl)-N,N-dimethyl-formamidine (220mg) and triazolyl aniline (113mg) in acetic acid (2ml) was heated to 125 for 2h. The cooled mixture was diluted with water and filtered under suction overnight. The resulting solid was sonicated in acetone (20ml) with warming and filtered. The solid was dried to give the title compound as a yellow solid (28mg).
$^1H$ NMR ($D_6$-DMSO) δ 3.86 (3H, s); 6.88 (1H, d); 7.28 (1H, d); 7.36 (1H, s); 7.82 (3H, m); 8.03 (2H, m); 8.15 (2H, m); 8.56 (1H, s); 8.69 (1H, s); 9.2 (2H, s); 9.98 (1H, s); LC-MS rt 2.14 m/z 411 ES+

Example 36: 2-Methoxy-5-[4-(4-[1,2,4]triazol-1-yl-phenylamino)-quinazolin-6-yl]-phenol

This compound may be prepared by the method in Example 35, the appropriate boronate being prepared from 5-bromo-2-methoxyphenol, itself prepared by Sandmeyer reaction (see JOC, 1993, 58, 1,42). The required boronic acid / boronate may be prepared by a palladium catalysed boronation using bis (pinacolato)borane or by temporary protection of the phenol ie with dihydropyran, followed by lithiation, reaction with trimethoxyborate followed hydrolysis and concomitant removal of the protecting group.

Example 37: {6-[3-Fluoro-4-(2-methoxy-ethoxy)-phenyl]-quinazolin-4-yl}-(2-methyl-4-[1,2,4]triazole-1-yl-phenyl)-amine

This compound may be prepared by the method outlined in Example 27, using the appropriate formamidine described as intermediate 22.

Example 38: HCV Replicon activity

Materials:

HCV replicon cell line

- 1b replicon (Huh.7) described in Science 285, 110-113.

  o Huh-9B: liver cell line with persistent bicistronic HCV genotype 1b coding sequence: [$I_{389}$lucubineo_3-3'_ET] includes firefly luciferase-ubiquitin-neomycin phosphotransferase fusion protein and EMCV-IRES driven non-structural HCV (NS3 to NS5B) coding sequence including cell culture adaptive mutations E1202G, T1280I and K1846T (Lohmann *et al,* 2001).

Method:

This assay is set up using all 96 wells of flat-bottomed 96-well plates. Plates are set up one day before addition of compounds. The assay then runs for 4 days with ELISA development taking place on the 5$^{th}$ day.

**Day 1**

Set up of Assay Plates

Exponentially growing Huh-9B monolayers are washed with sterile PBS to remove serum and treated with trypsin to detach cells from the flask.

Cells are suspended in growth media and counted using a haemocytometer. Duplicate 96 well plates are seeded with Huh-9B at a density of $10^4$ cells/well in a total volume of 100 μl/well of growth medium without antibiotics. as depicted below.

One of the plates is an opaque white 96-well plate used for IC50 determination based on the luciferase signal (referred as replicon plate), the other one is a clear 96-well plate used for a parallel determination of drug toxicity by methylene blue staining (referred as tox plate). Wells G12 and H12 of the tox plate are left without cells to use as buffer alone background reading.

Plates are then incubated at 37 °C in a 5 % $CO_2$ environment for 24 h to obtain a 90 % confluent cell monolayer.

**Day 2**

Addition of Compound and Compound Dilutions

Doubling dilutions of each compound are generated in a separate 96 high volume capacity round bottom plate to twice their initial concentration in the assay using growth medium without antibiotics.

Five compounds (C1 to C5) are tested on each assay plate as illustrated below plus a control compound that is also included in each plate.

Compounds are tested across an 8 point doubling dilution series. The initial dilution of each compound to be tested is 25 μM and 12.5 μM for the control compound.

DMSO only wells (A1 and A2) at 1 % provide the signal corresponding to maximal (100 %) luciferase detection. Previous optimization experiments showed negligible luciferase signal from non-replicon containing cells and control wells for background (unspecific) level of detection are not routinely included. The signal from the DMSO wells at 1 % (maximal signal) constitutes the assay window.

For each compound dilution on the 2 x 96-well dilution plate 100 μl are transferred using a multichannel pipette onto the replicon and tox plates mirror wells which contain 100 μl of medium to obtain the desired final concentration.

**Day 5**

Luciferase detection stage on the replicon plate

Media is tapped out from wells into Virkon and plates are washed once in warm PBS and tapped dry gently.

For each well 20 μl of lysis buffer is added by multichannel pipette. Lysates are stable at this point for several hours.

Luciferase assay buffer is placed it in the luminometer (Lmax, Molecular Devices).

The M injector is primed with 4x 300 μl of luciferase assay buffer. The plate to be analyzed is placed in the luminometer and 100 μl of luciferase assay buffer injected automatically into one well followed by 4 seconds integration read out.

After one second delay a second well is injected with 100 μl of luciferase assay buffer followed by 4 seconds integration read out and so forth until all 96 wells are analyzed.

Once the reading is finished the luminometer injection system is washed with deionised water.

The data is acquired using the SOFTmax for Lmax Pro software package.

Toxicity determination on the tox plate

Media is tapped out from wells into Virkon and plates tapped dry gently. To each well 100 μl of 0.5 % solution of methylene blue in 50 % methanol is added to all wells including blanks (G12 and H12). Plates are left at RT for a minimum of 1 h. Plates are then rinsed gently by immersing in a plastic box with water, tapped dry gently and left open until they are fully dry. Dye is solubilized adding 100 μl of 1 % lauroylsarcosine to each well and shaking for 1 h at 37 °C. Plates are read on the SpectraMax spectrophotometer at 620 nm wavelength using the SOFTmax Pro software package.

Results:

SOFTmax data files are exported as Excel or text files. A standard four parameters non-linear regression analysis of the data obtained from each compound is then used to calculate the IC50.

In the above analysis all replicate wells are meaned. The % of control is then calculated for each concentration point as a percentage of the DMSO control wells.

| | RepliconIC50 ***<5μM; **=5-20μM; *>20μM | Replicon TD50 **>25μM, *<25μM |
|---|---|---|
| Example No. | uM | uM |
| 1 | *** | * |
| 2 | ** | ** |
| 3 | *** | ** |
| 4 | ** | ** |
| 5 | *** | ** |
| 6 | * | ** |
| 7 | *** | ** |
| 8 | *** | ** |
| 9 | *** | ** |
| 10 | *** | * |
| 11 | *** | ** |
| 12 | *** | * |
| 13 | ** | * |
| 14 | ** | ** |
| 15 | *** | ** |
| 16 | *** | ** |
| 17 | *** | ** |
| 18 | *** | ** |
| 19 | *** | ** |
| 20 | *** | * |
| 21 | *** | ** |
| 22 | *** | * |
| 23 | *** | * |
| 24 | *** | ** |
| 25 | *** | * |
| 26 | *** | * |
| 27 | *** | * |
| 28 | ** | ** |
| 29 | ** | ** |
| 30 | *** | * |
| 31 | *** | ** |
| 32 | *** | * |

(continued)

| | RepliconIC50 ***<5μM; **=5-20μM; *>20μM | Replicon TD50 **>25μM, *<25μM |
|---|---|---|
| Example No. | uM | uM |
| 33 | *** | ** |
| 34 | *** | ** |
| 35 | | |
| 36 | | |
| 37 | | |

Example 39: Synergistic action between HCV replication inhibitors and human interferon αA

ELISA experiments were carried out on the combined effect of HCV replication inhibitor [6-(3,4-Dimethoxy-phenyl)-quinazolin-4-yl]-(4-[1,2,4]triazol-1-yl-phenyl)-amine with interferon αA (Sigma Hu-INF-α A order number 14276).

ELISA protocol as in Example 38

The IC50 for [6-(3,4-Dimethoxy-phenyl)-quinazolin-4-yl]-(4-[1,2,4]triazol-1-yl-phenyl)-amine was calculated for each background concentration of interferon αA. Similarly, the IC50 for interferon αA was calculated for each background concentration of [6-(3,4-Dimethoxy-phenyl)-quinazolin-4-yl]-(4-[1,2,4]triazol-1-yl-phenyl)-amine.

[6-(3,4-Dimethoxy-phenyl)-quinazolin-4-yl]-(4-[1,2,4]triazol-1-yl-phenyl)-amine had an ELISA IC50 of 0.2 μM against HCV replicon 1b.
Interferon αA had an ELISA IC50 of 6 u/ml against HCV replicon 1b.

In combination, at concentrations of [6-(3,4-Dimethoxy-phenyl)-quinazolin-4-yl]-(4-[1,2,4]triazol-1-yl-phenyl)-amine below its IC50, the IC50 of interferon αA was reduced from 6u/ml to at least 0.008 u/ml. At concentrations of interferon αA below its IC50, the IC50 of [6-(3,4-Dimethoxy-phenyl)-quinazolin-4-yl]-(4-[1,2,4]triazol-1-yl-phenyl)-amine is reduced from 0.2μM to at least 0.067μM.

The fractional inhibitory concentratin (FIC) can be used to identify a synergistic interaction.

$$FIC = \frac{\text{Lowest IC50 Cpd A}^{\text{COMBINATION}}}{\text{IC50 Cpd A}^{\text{ALONE}}} + \frac{\text{Lowest IC50 Cpd B}^{\text{COMBINATION}}}{\text{IC50 Cpd B}^{\text{ALONE}}}$$

where FIC value
<0.5 SYNERGY
0.5 - 1.0 ADDITION
1.0 - 2.0 INDIFFERENCE
>2.0 ANTAGONISM

$$FIC = \frac{0.008\text{u/ml}}{6\text{u/ml}} + \frac{0.067\text{uM}}{0.2\mu\text{M}} = \underline{0.336}$$

## Claims

1. A quinazoline derivative having formula (Ib) or a pharmaceutically acceptable salt thereof:

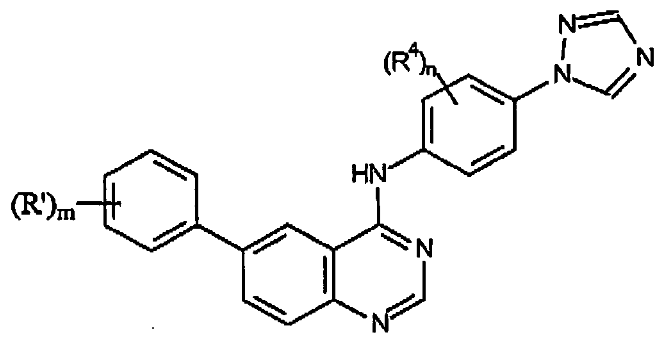

(Ib)

wherein:

- each $R^4$ is the same or different and represents halogen, $C_{1-4}$ alkyl or $C_{1-4}$ alkoxy;
- n is 0, 1 or 2;
- each $R^{/}$ is the same or different and represents halogen, $C_{1-4}$ alkyl or $C_{1-4}$ alkoxy; and
- m is 0, 1 or 2.

**2.** A quinazoline derivative having formula (Ic), or a pharmaceutically acceptable salt thereof:

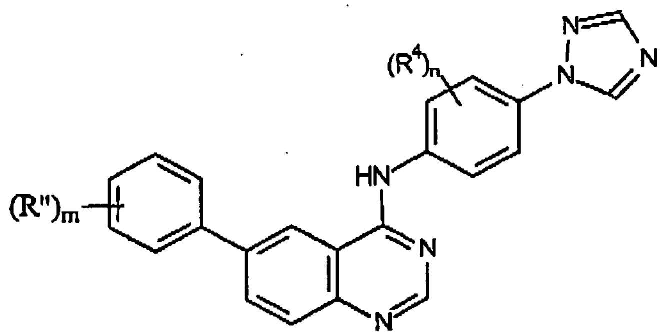

(Ic)

wherein:

- each $R^4$ is the same or different and represents halogen, $C_{1-4}$ alkyl or $C_{1-4}$ alkoxy;
- m is 1;
- n is 0, 1 or 2; and
- $R^{//}$ is $-A^{/}$, $-Het-A^{/}$, $-L-A^{/}$, $-Het-L-A^{/}$, $-L-Het-A^{/}$, $-Het-L-Het^{/}-A^{/}$ or $Het-L-Het^{/}-L^{/}$ wherein:
- A' represents a phenyl, 5- to 10-membered heteroaryl, 5- to 10-membered heterocyclyl or $C_{3-6}$ carbocyclyl group which is optionally fused to a further phenyl, 5- to 10-membered heteroaryl, 5- to 10-membered heterocyclyl or $C_{3-6}$ carbocyclyl group;
- each Het and Het' is the same or different and represents -O-, -S- or -NR'-where R' is hydrogen or $C_{1-4}$ alkyl;
- each L is the same or different and represents $C_{1-4}$ alkylene; and
- each L' is the same or different and represents hydrogen or a $C_{1-4}$ alkyl, $C_{1-4}$ haloalkyl, $C_{1-4}$ hydroxyalkyl or $C_{1-4}$ aminoalkyl group,

the phenyl, heteroaryl, heterocyclyl and carbocyclyl moieties in A' being unsubstituted or substituted by 1, 2 or 3 unsubstituted substituents which are the same or different and are selected from halogen atoms and $C_{1-4}$ alkyl, $C_{1-4}$ alkoxy, $C_{1-4}$ haloalkyl, $C_{1-4}$ haloalkoxy, hydroxy, cyano, nitro and -NR'R'', wherein each R' and R'' is the same or different and represents hydrogen or $C_{1-4}$ alkyl.

**3.** A compound according to claim 2, wherein each A' is the same or different and is a non-fused phenyl, 5- to 6-membered heteroaryl or 5- to 6- membered heterocyclyl.

**4.** A compound according to claim 2 or 3, wherein the phenyl, heteroaryl, heterocyclyl and carbocyclyl moieties in the

A' groups are unsubstituted or substituted with 1 or 2 unsubstituted substituents which are the same or different and are selected from halogen, hydroxy, $C_{1-4}$ alkyl and $C_{1-4}$ alkoxy.

**5.** A compound according to any one of the claims 2 to 4, wherein each A' is the same or different and is a non-fused 5- to 6- membered heteroaryl or heterocyclyl group which is unsubstituted or substituted with 1 or 2 unsubstituted substituents which are the same or different and are selected from halogen, $C_{1-4}$ alkyl and $C_{1-2}$ alkoxy.

**6.** A compound according to any one of the preceding claims, wherein n is 0 or 1.

**7.** A quinazoline derivative of the formula (Ib) or (Ic), as defined in claim 1 or 2, for use in treating the human or animal body.

**8.** A pharmaceutical composition which comprises a quinazoline derivative of the formula (Ib) or (Ic), as defined in claim 1 or 2, or a pharmaceutically acceptable salt thereof, and a pharmaceutically acceptable carrier or diluent.

**9.** Use of a quinazoline derivative of the formula (Ib) or (Ic), as defined in claim 1 or 2, or a pharmaceutically acceptable salt thereof, in the manufacture of a medicament for use in treating or preventing a flaviviridae infection.

**10.** Use according to claim 9,wherein the flavivirdae infection is a hepacivirus infection.

**11.** Use according to claim 10, wherein the hepacivirus infection is an infection by a hepatitis C virus.

**12.** Use according to claim 11, wherein the medicament further comprises (a) interferon or a derivative thereof and/or (b) ribavirin or a derivative thereof.

**13.** Use according to claim 12 wherein the interferon derivative is PEG-interferon and/or the ribavirin derivative is viramidine.

**14.** A product containing:

(a) a quinazoline derivative of the formula (Ib) or (Ic), as defined in claim 1 or 2, or a pharmaceutically acceptable salt thereof; and
(b) interferon or an interferon derivative and/or ribavirin or a ribavirin derivative;
for simultaneous, separate or sequential use in the treatment of the human or animal body.

**15.** A pharmaceutical composition which comprises (a) a quinazoline derivative of the formula (Ib) or (Ic) as defined in claim 1 or 2, or a pharmaceutically acceptable salt thereof, and (b) interferon or an interferon derivative, together with a pharmaceutically acceptable carrier or diluent.

**16.** Use of a quinazoline derivative of formula (Ib) or (Ic), as defined in claim 1 or 2, or a pharmaceutically acceptable salt thereof, in the manufacture of a medicament for use in treating or preventing an HCV infection, by co-administration with an interferon or interferon derivative.

**17.** A compound according to Claim 1, which is [6-(3,4-dimethoxy-phenyl)-quinazolin-4-yl]-(4-[1,2,4]triazol-1-yl-phenyl)-amine, or a pharmaceutically acceptable salt thereof,

## Patentansprüche

**1.** Chinazolinderivat der Formel (Ib) oder ein pharmazeutisch annehmbares Salz davon:

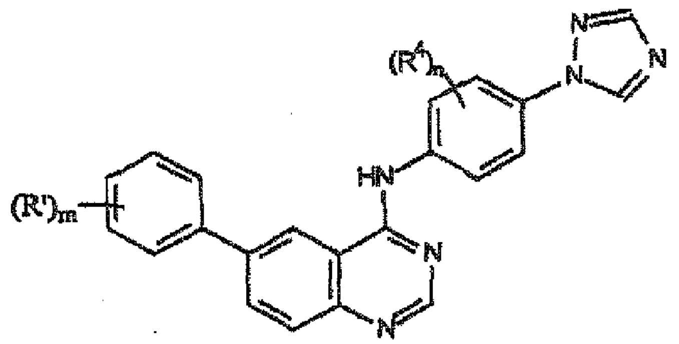

(Ib)

worin:

- $R^4$ jeweils gleich oder verschieden ist und für Halogen, $C_{1-4}$-Alkyl oder $C_{1-4}$-Alkoxy steht;
- n für 0, 1 oder 2 steht;
- R' jeweils gleich oder verschieden ist und für Halogen, $C_{1-4}$-Alkyl oder $C_{1-4}$-Alkoxy steht und
- m für 0, 1 oder 2 steht.

**2.** Chinazolinderivat der Formel (Ic) oder ein pharmazeutisch annehmbares Salz davon:

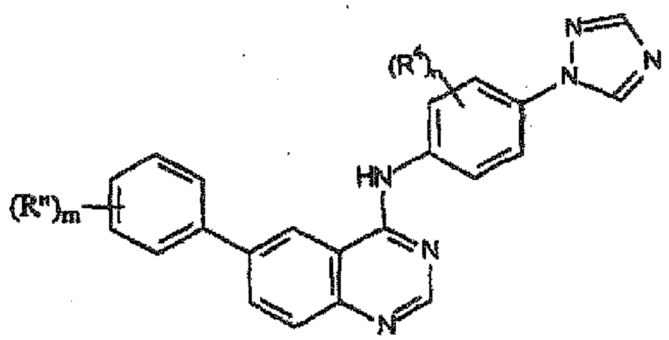

(Ic)

worin:

- $R^4$ jeweils gleich oder verschieden ist und für Halogen, $C_{1-4}$-Alkyl oder $C_{1-4}$-Alkoxy steht;
- m für 1 steht;
- n für 0, 1 oder 2 steht und
- R" für -A', -Het-A', -L-A', -Het-L-A', -L-Het-A', -Het-L-Het'-A' oder -Het-L-Het'-L' steht, wobei:
- A' für eine Phenyl-, 5- bis 10-gliedrige Heteroaryl-, 5- bis 10-gliedrige Heterocyclyl- oder $C_{3-6}$-Carbocyclylgruppe steht, die gegebenenfalls an eine weitere Phenyl-, 5- bis 10-gliedrige Heteroaryl-, 5- bis 10-gliedrige Heterocyclyl- oder $C_{3-6}$-Carbocyclylgruppe anelliert ist;
- Het und Het' jeweils gleich oder verschieden sind und für -O-, -S- oder -NR'- stehen, wobei R' für Wasserstoff oder $C_{1-4}$-Alkyl steht;
- L jeweils gleich oder verschieden ist und für $C_{1-4}$-Alkylen steht und
- L' jeweils gleich oder verschieden ist und für Wasserstoff oder eine $C_{1-4}$-Alkyl-, $C_{1-4}$-Halogenalkyl-, $C_{1-4}$-Hydroxyalkyl- oder $C_{1-4}$-Aminoalkylgruppe steht;

wobei die Phenyl-, Heteroaryl-, Heterocyclyl- und Carbocyclylgruppierungen in A' unsubstituiert oder durch 1, 2 oder 3 unsubstituierte Substituenten, die gleich oder verschieden sind und unter Halogenatomen und $C_{1-4}$-Alkyl, $C_{1-4}$-Alkoxy, $C_{1-4}$-Halogenalkyl, $C_{1-4}$-Halogenalkoxy, Hydroxy, Cyano, Nitro und -NR'R" ausgewählt sind, substituiert sind, wobei R' und R" jeweils gleich oder verschieden sind und für Wasserstoff oder $C_{1-4}$-Alkyl stehen.

**3.** Verbindung nach Anspruch 2, wobei A' jeweils gleich oder verschieden ist und für ein nichtanelliertes Phenyl, 5- bis 6-gliedriges Heteroaryl oder 5- bis 6-gliedriges Heterocyclyl steht.

4. Verbindung nach Anspruch 2 oder 3, wobei die Phenyl-, Heteroaryl-, Heterocyclyl- und Carbocyclylgruppierungen in den Gruppen A' unsubstituiert oder durch 1 oder 2 unsubstituierte Substituenten, die gleich oder verschieden sind und unter Halogen, Hydroxy, $C_{1-4}$-Alkyl und $C_{1-4}$-Alkoxy ausgewählt sind, substituiert sind.

5. Verbindung nach einem der Ansprüche 2 bis 4, wobei A' jeweils gleich oder verschieden ist und für eine nicht-anellierte 5- bis 6-gliedrige Heteroaryl- oder Heterocyclylgruppe, die unsubstituiert oder durch 1 oder 2 unsubstituierte Substituenten, die gleich oder verschieden sind und unter Halogen, $C_{1-4}$-Alkyl und $C_{1-2}$-Alkoxy ausgewählt sind, substituiert ist, steht.

6. Verbindung nach einem der vorhergehenden Ansprüche, wobei n für 0 oder 1 steht.

7. Chinazolinderivat der Formel (Ib) oder (Ic) gemäß Anspruch 1 bzw. 2 zur Verwendung bei der Behandlung des menschlichen oder tierischen Körpers.

8. Pharmazeutische Zusammensetzung, die ein Chinazolinderivat der Formel (Ib) oder (Ic) gemäß Anspruch 1 bzw. 2 oder ein pharmazeutisch annehmbares Salz davon und einen pharmazeutisch annehmbaren Träger oder ein pharmazeutisch annehmbares Verdünnungsmittel enthält.

9. Verwendung eines Chinazolinderivats der Formel (Ib) oder (Ic) gemäß Anspruch 1 bzw. 2 oder eines pharmazeutisch annehmbaren Salzes davon bei der Herstellung eines Medikaments zur Verwendung bei der Behandlung oder Prävention einer Flaviviridae-Infektion.

10. Verwendung nach Anspruch 9, wobei es sich bei der Flaviviridae-Infektion um eine Hepacivirus-Infektion handelt.

11. Verwendung nach Anspruch 10, wobei es sich bei der Hepacivirus-Infektion um eine Infektion durch ein Hepatitis-C-Virus handelt.

12. Verwendung nach Anspruch 11, wobei das Medikament ferner (a) Interferon oder ein Derivat davon und/oder (b) Ribavirin oder ein Derivat davon enthält.

13. Verwendung nach Anspruch 12, wobei es sich bei dem Interferonderivat um PEG-Interferon und/oder bei dem Ribavirinderivat um Viramidin handelt.

14. Produkt, enthaltend:

    (a) ein Chinazolinderivat der Formel (Ib) oder (Ic) gemäß Anspruch 1 bzw. 2 oder ein pharmazeutisch annehmbares Salz davon und
    (b) Interferon oder ein Interferonderivat und/oder Ribavirin oder ein Ribavirinderivat;
    zur gleichzeitigen, getrennten oder aufeinanderfolgenden Verwendung bei der Behandlung des menschlichen oder tierischen Körpers.

15. Pharmazeutische Zusammensetzung, die (a) ein Chinazolinderivat der Formel (Ib) oder (Ic) gemäß Anspruch 1 bzw. 2 oder ein pharmazeutisch annehmbares Salz davon und (b) Interferon oder ein Interferonderivat zusammen mit einem pharmazeutisch annehmbaren Träger oder Verdünnungsmittel enthält.

16. Verwendung eines Chinazolinderivats der Formel (Ib) oder (Ic) gemäß Anspruch 1 bzw. 2 oder eines pharmazeutisch annehmbaren Salzes davon bei der Herstellung eines Medikaments zur Verwendung bei der Behandlung oder Prävention einer HCV-Infektion durch gemeinsame Verabreichung mit Interferon oder einem Interferonderivat.

17. Verbindung nach Anspruch 1, bei der es sich um [6-(3,4-Dimethoxyphenyl)chinazolin-4-yl](4-[1,2,4]-triazol-1-ylphenyl)amin handelt, oder ein pharmazeutisch annehmbares Salz davon.

## Revendications

1. Dérivé de quinazoline de formule (Ib) ou l'un de ses sels de qualité pharmaceutique :

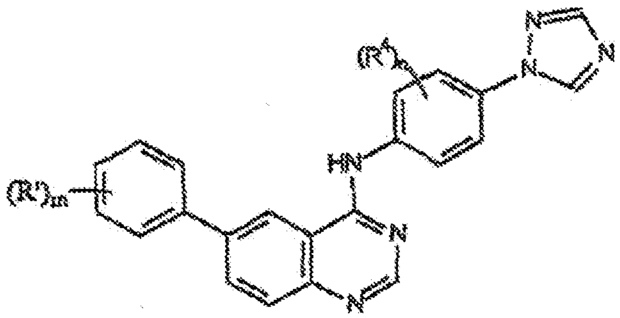

(Ib)

où :

- chacun des radicaux $R^4$ est identique ou différent et représente un atome d'halogène, un groupement alkyle en $C_{1-4}$ ou un groupement alkoxy en $C_{1-4}$ ;
- n est égal à 0, 1 ou 2 ;
- chacun des radicaux R' est identique ou différent et représente un atome d'halogène, un groupement alkyle en $C_{1-4}$ ou un groupement alkoxy en $C_{1-4}$ ; et
- m est égal à 0, 1 ou 2.

**2.** Dérivé de quinazoline de formule (Ic) ou l'un de ses sels de qualité pharmaceutique :

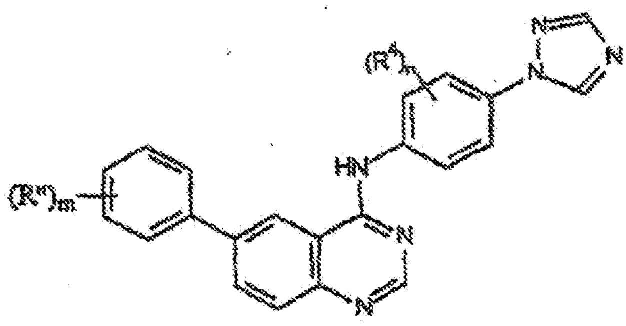

(Ic)

où :

- chacun des radicaux $R^4$ est identique ou différent et représente un atome d'halogène, un groupement alkyle en $C_{1-4}$ ou un groupement alkoxy en $C_{1-4}$ ;
- m est égal à 1 ;
- n est égal à 0, 1 ou 2 ; et
- R" représente -A', -Het-A', -L-A', -Het-L-A', -L-Het-A', -Het-L-Het'-A' ou -Het-L-Het'-L' où :
- A' représente un groupement phényle, hétéroaryle comportant entre 5 et 10 chaînons, hétérocyclyle comportant entre 5 et 10 chaînons ou carbocyclyle en $C_{3-6}$ éventuellement fusionné à un groupement phényle, hétéroaryle comportant entre 5 et 10 chaînons, hétérocyclyle comportant entre 5 et 10 chaînons ou carbocyclyle en $C_{3-6}$ supplémentaire ;
- chacun des groupements Het et Het' est identique ou différent et représente -O-, -S- ou -NR'-, R' représentant un atome d'hydrogène ou un groupement alkyle en $C_{1-4}$ ;
- chacun des groupements L est identique ou différent et représente un groupement alkylène en $C_{1-4}$ ; et
- chacun des groupements L'est identique ou différent et représente un atome d'hydrogène ou un groupement alkyle en $C_{1-4}$, halogénoalkyle en $C_{1-4}$, hydroxyalkyle en $C_{1-4}$ ou aminoalkyle en $C_{1-4}$,

les groupements phényle, hétéroaryle, hétérocyclyle et carbocyclyle de A' étant éventuellement substitués par 1, 2 ou 3 substituants non substitués identiques ou différents choisis parmi les atomes d'halogène et les groupements alkyle en $C_{1-4}$, alkoxy en $C_{1-4}$, halogénoalkyle en $C_{1-4}$, halogénoalkoxy en $C_{1-4}$, hydroxy, cyano, nitro et -NR'R", chacun des groupements R' et R" étant identique ou différent et représentant un atome d'hydrogène ou un groupement alkyle en $C_{1-4}$.

**3.** Composé conforme à la revendication 2, où chacun des groupements A' est identique ou différent et représente un

groupement non fusionné phényle, hétéroaryle comportant 5 à 6 chaînons ou hétérocyclyle comportant 5 à 6 chaînons.

4. Composé conforme à la revendication 2 ou 3, où les groupements phényle, hétéroaryle, hétérocyclyle et carbocyclyle des groupements A' sont éventuellement substitués par 1 ou 2 substituants non substitués identiques ou différents choisis parmi les atomes d'halogène et les groupements hydroxy, alkyle en $C_{1-4}$ et alkoxy en $C_{1-4}$.

5. Composé conforme à l'une quelconque des revendications 2 à 4, où chacun des groupements A' est identique ou différent et représente un groupement non fusionné hétérocyclyle ou hétéroaryle comportant entre 5 et 6 chaînons, éventuellement substitué par 1 ou 2 substituants non substitués identiques ou différents choisis parmi les atomes d'halogène et les groupements alkyle en $C_{1-4}$ et alkoxy en $C_{1-2}$.

6. Composé conforme à l'une quelconque des revendications précédentes, où n est égal à 0 ou à 1.

7. Dérivé de quinazoline de formule (Ib) ou (Ic) conforme à la revendication 1 ou 2, pour emploi dans le traitement de l'organisme humain ou animal.

8. Composition pharmaceutique comprenant un dérivé de quinazoline de formule (Ib) ou (Ic) conforme à la revendication 1 ou 2, ou l'un de ses sels de qualité pharmaceutique, et un vecteur ou diluant de qualité pharmaceutique.

9. Emploi d'un dérivé de quinazoline de formule (Ib) ou (Ic) conforme à la revendication 1 ou 2, ou de l'un de ses sels de qualité pharmaceutique, dans la fabrication d'un médicament destiné au traitement prophylactique ou thérapeutique d'une infection par des flavivirus.

10. Emploi conforme à la revendication 9, où l'infection par des flavivirus est une infection par des hépacivirus.

11. Emploi conforme à la revendication 10, ou l'infection par des hépacivirus est une infection par un virus de l'hépatite C.

12. Emploi conforme à la revendication 11, où le médicament comprend en outre (a) un interféron ou l'un de ses dérivés et/ou (b) la ribavirine ou l'un de ses dérivés.

13. Emploi conforme à la revendication 12, ou le dérivé d'interféron est un PEG-interféron et/ou le dérivé de ribavirine est la viramidine.

14. Produit incluant :

(a) un dérivé de quinazoline de formule (Ib) ou (Ic) conforme à la revendication 1 ou 2, ou l'un de ses sels de qualité pharmaceutique ; et
(b) un interféron ou l'un de ses dérivés et/ou la ribavirine ou l'un de ses dérivés ;
pour emploi simultané, séparé ou séquentiel dans le traitement de l'organisme humain ou animal.

15. Composition pharmaceutique comprenant (a) un dérivé de quinazoline de formule (Ib) ou (Ic) conforme à la revendication 1 ou 2, ou l'un de ses sels de qualité pharmaceutique, et (b) un interféron ou l'un de ses dérivés, avec un vecteur ou diluant de qualité pharmaceutique.

16. Emploi d'un dérivé de quinazoline de formule (Ib) ou (Ic) conforme à la revendication 1 ou 2, ou de l'un de ses sels de qualité pharmaceutique, dans la fabrication d'un médicament destiné au traitement prophylactique ou thérapeutique d'une infection par un VHC, par co-administration avec un interféron ou l'un de ses dérivés.

17. Composé conforme à la revendication 1, qui est la [6-(3,4-diméthoxy-phényl)-quinazolin-4-yl]-(4-[1,2,4]triazol-1-ylphényl)-amine, ou l'un de ses sels de qualité pharmaceutique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9802434 A
- WO 05105761 A
- GB 2187731 A, M Elliott, N Janes & B Khambay
- WO 9609294 A1
- WO 0305549 A


### Non-patent literature cited in the description


- *Clinical Microbiology Reviews,* January 2000, 67-82
- **A. Rosowsky ; H. Chen.** *J. Org. Chem.,* 2001, vol. 66, 7522-7526
- **Meyers ; Snyder.** *J. Org. Chem,* 1993, vol. 58 (1), 42
- **C. Zhou ; R. C. Larock.** *Journal of Organic Chemistry,* vol. 70 (10), 3765
- *JOC,* 1993, vol. 58 (1), 42
- *Science,* vol. 285, 110-113